# EUROPEAN PATENT SPECIFICATION

(11) **EP 2 558 859 B1**
(45) Date of publication and mention of the grant of the patent: **27.09.2017**
(21) Application number: 11769148.5
(22) Date of filing: 15.04.2011
(51) Int. Cl.: C12Q 1/68, G01N 33/53, C12M 1/34

(54) **THREE DIMENSIONSAL MICROARRAYS**
DREI-DIMENSIONAL MIKROARRAYS
MICRO-RESEAUX TRI-DIMENSIONNEL

(30) Priority: 31.05.2010 AU 2010902365; 15.04.2010 AU 2010901595
(43) Date of publication of application: 20.02.2013
(73) Proprietor: Digital Sensing Limited, Auckland 0745 (NZ)
(72) Inventor: HAYNES, Andrew, North Shore, Auckland 0745 (NZ); PARTRIDGE, Ashton, Cyril, North Shore, Auckland 0745 (NZ); WU, Yinqiu, North Shore, Auckland 0745 (NZ)
(74) Representative: Appleyard Lees IP LLP
(86) International application number: PCT/NZ2011/000052
(87) International publication number: WO 2011/129710

(56) References cited:
- WO-A2-03/064997
- KR-A- 20100 114 238
- US-A1- 2002 182 603
- US-A1- 2002 187 249
- US-A1- 2005 042 866
- US-A1- 2008 119 372
- US-A1- 2009 042 741
- US-A1- 2010 075 865
- PETERSHANS A ET AL: "TOF-SIMS analysis of structured surfaces biofunctionalized by a one-step coupling of a spacer-linked GRGDS peptide", JOURNAL OF COLLOID AND INTERFACE SCIENCE, ACADEMIC PRESS, NEW YORK, NY, US, vol. 341, no. 1, 1 January 2010 (2010-01-01), pages 30-37, XP026987167, ISSN: 0021-9797 [retrieved on 2009-09-16]
- OILLIC, C. ET AL.: 'Silicon nanostructures for DNA biochip applications' MATERIALS SCIENCE AND ENGINEERING C vol. 27, 2007, pages 1500 - 1503, XP022190539

## Description

### Technical Field

The invention relates to the development of three-dimensional microarrays for use in detection/sensing applications with high sensitivity and selectivity.

In particular the invention relates generally to the detection of compound(s) or target analytes in a sample, particularly but not exclusively to determining whether a nucleic acid comprising a specific sequence of bases is present within a sample, quantifying the number of the specific nucleic acid base sequences within a sample, determining the presence and/or extent of methylation in the promoter region for a particular gene, and the effects of various factors, including environment, diet or medications, on nucleic acid methylation.

### Background Art

There is increasing need for fast, accurate and cost effective methods of detecting and identifying various target analytes, including but not limited to, molecules or proteins including antibodies, across a range of industries. In particular, there is increased demand for tests that can, if desired, be carried out away from standard laboratory settings by non technical personnel. While sensor technology is a rapidly expanding area of technology, a number of problems remain which prevent on-site testing and accurate detection beyond certain sensitivity limits. These problems include: the complicated nature of the reading instruments and/or sensing processes, meaning considerable training and expertise is often required; low sensitivity sensing technology that requires concentrations of the target analyte before detection is possible; the large size and correspondingly non-portable nature of the sensing instrumentation, which need to be contained within a laboratory; in the case of micro organisms the time required for the growth of detectable concentrations of the target species; the surface areas on which reactions or binding under study can occur and the correspondingly low surface area availability; carrying out measurements is often difficult; and finally the requirement for reference standards against which any measurements obtained can be referenced.

Nucleic acid (NA) detection and base pair determination, and the determination of the presence or extent of methylation in the promoter region of a gene could all benefit from improved sensor technology. NA detection and base pair determination typically requires the use of Polymerase Chain Reaction (PCR), a process used to amplify a few copies of nucleic acid by several orders of magnitude using enzymatic replication. The subsequent methodology used to analyse the sample depends on the quantity of nucleic acid in the sample and the detail required in the analysis. These methodologies therefore range in complexity from simple electrophoretic gels, which give sample to sample comparison, to the more complicated mass spectroscopic techniques, which give details down to the atomic level.

DNA methylation is the covalent addition of a methyl group to the 5-carbon of Cytosine in a CpG dinucleotide (the region of DNA where a Cytosine nucleotide is linked to a Guanine nucleotide through a phosphodiester bond). The covalent addition reaction is catalysed by DNA methyltransferase. DNA methylation affects cell function by altering gene expression without changing the DNA sequence. The alterations can also be heritable during cell division.

Transcription of a gene requires the attachment of RNA polymerase (which carries out the replication process) to a promoter (or epigenetic) region. The promoter region of a gene contains specific DNA sequences and response elements. Promoter regions may contain clusters of CpG dinucleotides which may be referred to as CpG islands or regions. If the Cytosine base(s) in one or more CpG dinucleotides is/are methylated the promoter region is no longer available, preventing transcription of the gene. Therefore methylated promoter regions cannot be accessed, while non-methylated promoter regions can be. DNA methylation has therefore been found to play an important role in both the development and normal function of organisms and the development of disease, and is consequently the subject of intense research.

Much research that correlates DNA with various conditions (e.g. diseases, phenotypes etc) centers around the role that DNA methylation plays in the promoter regions of the DNA strand. This is because epigenetic alterations have been shown to be common in cancer and typically involve hypermethylation and hypomethylation of DNA. Hypermethylation refers to an increase in the extent of methylation of CpG regions. This in turn results in heritable transcriptional silencing, and where tumour suppressor genes are silenced, cancerous cells can result. Tumour suppressor genes provide the code for anti-proliferation signals and proteins responsible for suppressing mitosis and cell growth. By comparison, hypomethylation refers to a decrease in methylation of other regions of the genome. Hypomethylation typically occurs in repetitive DNA that is normally heavily methylated, resulting in increased transcription and an elevated mutation rate due to the activation of otherwise silenced gene expression.

DNA methylation changes in cancer, particularly hypermethylation of CpG regions, has been found to occur relatively early in the development of cancer. Therefore DNA methylation could act as an important biomarker for the detection of diseases such as cancer. Furthermore, because the gene sequence remains unchanged following methylation, individual genes which have been silenced by methylation remain intact and can therefore be reactivated by small molecule inhibitors of DNA methyltransferase's.

Recent research has suggested that the methylation of promoter regions can be affected by a variety of factors, including diet and environmental factors Mass spectroscopy is typically employed for determining the existence of methylation and quantifying changes in DNA methylation, however this involves a number of preparatory steps (for example, the replication of DNA) and is expensive and time consuming.

### Object of the Invention

It is an object of the invention to provide three-dimensional microarrays for use in detection/sensing applications with high sensitivity and selectivity. It is a further or alternate object of the invention to provide a method for determining whether or not a particular molecule(s) or compound(s) is present within a sample and/or have been modified in any way. It is a further or alternate object of the invention to provide a method for determining whether a nucleic acid comprising a specific sequence of bases is present in a sample. It is a further or alternate object of the invention to enable the detection of a nucleic acid comprising a specific base sequence without the need for pre-concentration using PCR. It is a further or alternate object of the invention to provide a useful method to quantify by counting the number of nucleic acids comprising a specific nucleic acid base sequence within a sample. It is a further or alternate object to provide a method of determining the presence and/or extent of methylation in the promoter region of a gene and/or the effect of factors, such as environment, diet, or medication, on nucleic acid methylation. It is a further or alternate object of the invention to at least to provide the public with a useful choice.

### Summary of the Invention

In a first aspect the invention provides a method for determining the presence of a target compound(s) of interest within a sample, the method including the steps of:
a) Providing the three-dimensional microarray of any one of claims 1-11;
b) Passing at least part of the sample over the array; and
c) Determining the presence of a target compound(s) of interest by detection of a detectable response to the attachment of the target compound(s) to the sensory agent(s).

Preferably a signal entity capable of providing a detectable response is attached to the target compound to be detected in the sample to form a mixed sample, and the sensor response given by the sensory agent(s) in the defined functionalized areas is provided by attachment of the signal entity to the sensory agent(s).

Preferably the compound to which the signal entity is attached is a synthesized complimentary copy of a single strand of a nucleic acid.

Preferably the signal entity is a chemical, biological, or physical entity which is capable of providing a detectable signal or response.

Preferably the signal entity is a particle and is selected from a coloured microbead, a fluorescent microbead, a magnetic microbead, or a light blocking microbead.

Preferably the particle is a polymer microbead including but not limited to Polystyrene beads, PMMA beads and PET beads.

Preferably the detectable sensor response is selected from colour, fluorescence, magnetic or light blocking.

Preferably the detectable sensor response is capable of being read by digital counting, weight measurements, fluorescence, optical, and/or electrical means.

Preferably the detectable sensor response results in any one or a combination of quantitative/qualitative, fluorescence, optical or colour metric measurements.

Preferably the detectable sensor response includes visual responses, spectrophotometric responses, fluorescent techniques, potentiometric or galvanostatic responses, magnetic light refraction, heat, frequency and digital responses.

Preferably the detectable sensor response is digital.

Preferably the detectable sensor response results in quantitative or qualitative measurements.

Preferably the surface structures are millimeter to nanometer sized surface structures which are substantially identical and uniformly separated from each other. Alternatively, the surface structures are randomly ordered on the surface of the microarray.

Preferably the surface structures take the form of cones or ridges.

Preferably the defined functionalized area of each surface structure is the tip of the said cones or ridges.

Preferably the tip of the surface structure is between about 1 nm and 1000 micron.

Preferably the tip of the surface structure is between about 1 micron and about 20 micron in diameter.

Preferably the tip of the surface structure is between about 5 micron and about 15 micron in diameter.

Preferably the tip of the surface structure is the same size or smaller than the size of the signal entity.

Preferably the tips of the surface structures are separated from each other by about 5 nm to about 20 micron spacing.

Preferably the tips of the surface structures are separated from each other by about 1 to about 20 micron spacing.

Preferably there are between about 250,000 and about 1 billion tips per cm².

Preferably there are about 250,000 tips per cm² at a 10 µm resolution.

Preferably the microarray includes a base material and is formed from a plastics material, including PMMA, PET and PS or metals such as aluminium or ceramics or oxides or silicon or a photoresist.

Preferably the microarray is formed from a polymer substrate.

Preferably a patterned layer is attached to or formed onto the underside of the base material to disperse light across the surface where light is passed through the microarray for measurement purposes.

Preferably the three dimensional microarray is formed by etching, lithograph processes, hot embossing, nano-embossing and injection molding or by the Continuous Forming Technology processes (as described in WO2007/058548).

Preferably the defined areas are functionalized using NH₂ or COOH functional groups.

Preferably one or more single strands of nucleic acid comprising a promoter region or one more single strands of nucleic acid comprising a specific base sequence of interest are attached to the NH₂ or COOH functional groups.

Preferably a linker group is attached to the NH₂ or COOH functional groups.

Preferably the linker group is a covalent linker group.

Preferably the sensory agent is attached to the linker group.

Preferably the sensory agent is attached directly to the NH₂ or COOH functional groups.

Preferably the linker groups are selected from aliphatic compounds, PEG molecules and polymers, proteins or DNA chains.

Alternatively the sensory agent is attached directly to the tips of the surface structures through a linker group.

Preferably the sensory agents are biological recognition groups or binding agents.

Preferably the sensory agent/target compound are biological bindings or recognition groups selected from antibody/antigen, DNA/DNA, DNA/protein, protein/protein, protein/receptor, cell/protein and cell/DNA binding partners.

Preferably the microarray includes a plurality of sensory agents, each sensory agent forming a section of the microarray.

Preferably each defined functionalized area includes a plurality of sensory agents.

Preferably the microarray is coated between the defined functionalized areas with an inert material.

Preferably, before coating, the microarray is treated with a thiol, protein or PEG material to ensure coating adhesion.

Preferably the inert material is selected from gold or silver or chromium, a polymer or an oil.

Preferably the inert material employed for coating purposes is a combination of any two metals selected from gold, silver or chromium.

Preferably the thickness of the inert coating ranges from sub nm to µm to mm measurements.

Preferably the inert material is applied using evaporation, painting, deposition, sputtering, plasma treatment, spray coating, dip coating, or spin coating.

Preferably the microarray includes a secondary inert coating.

Preferably the secondary inert coating is a thiol compound.

Preferably the sample is a biological sample, including but not limited to, a tissue sample, a fluid sample, or an oral swab. In one preferred embodiment, the sample is a biological sample comprising nucleic acid. In another embodiment, the sample is a biological sample comprising a micro-organism, a peptide or protein, and/or an antibody.

In a second aspect the invention provides a three-dimensional microarray for use in determining the presence of a target compound(s) of interest within a sample, the microarray including:
a) base material including a plurality of surface structures forming part of and protruding from the base material;
b) the plurality of surface structures including sensory agent(s) capable of attachment to the target compound(s) of interest within the sample, the sensory agent(s) being included on defined functionalized areas on the tops of each surface structure;
and wherein the microarray is coated between the defined functionalized areas with an inert material to minimize non-specific binding.

Preferably a signal entity capable of providing a detectable response is attached to the target compound to be detected in the sample to form a mixed sample, and the sensor response given by the sensory agent(s) in the defined functionalized areas is provided by attachment of the signal entity to the sensory agent(s).

Preferably the detectable sensor response is selected from colour, fluorescence, magnetic or light blocking.

Preferably the detectable sensor response is capable of being read by digital counting, weight measurements, fluorescence, optical, and/or electrical means.

Preferably the detectable sensor response results in any one or a combination of quantitative/qualitative, fluorescence, optical or colour metric measurements.

Preferably the detectable sensor response is digital.

Preferably the detectable sensor response results in quantitative or qualitative measurements.

Preferably the surface structures are millimeter to nanometer sized surface structures which are substantially identical and uniformly separated from each other.

Alternatively the surface structures are randomly ordered on the surface of the microarray.

Preferably the surface structures take the form of cones or ridges.

Preferably the defined functionalized area of each surface structure is the tip of the said cones or ridges.

Preferably the defined functionalized area on the surface structure is between about 1 nm and 1000 micron.

Preferably the defined functionalized area on the surface structure is between about 1 micron and about 20 micron in diameter.

Preferably the defined functionalized area on the surface structure is between about 5 micron and about 15 micron in diameter.

Preferably the tip of the surface structure is the same size or smaller than the size of the signal entity.

Preferably the defined functionalized areas on the surface structures are separated from each other by about 5 nm to about 20 micron spacing.

Preferably the defined functionalized areas on the surface structures are separated from each other by about 1 to about 20 micron spacing.

Preferably there are between about 250,000 and about 1 billion defined functionalized areas per cm².

Preferably there are about 250,000 defined functionalized areas per cm² at a 10 µm resolution.

Preferably the microarray includes a base material and is formed from a plastics material, metal, ceramics or oxides or silicon or a photoresist.

Preferably the plastics material is selected from PMMA, PET and PS.

Preferably the metal is aluminium.

Preferably the microarray is formed from a polymer substrate.

Preferably the base material has a thickness of between about 500 microns and about 2 mm.

Preferably a patterned layer is attached to or formed onto the underside of the base material to disperse light across the surface where light is passed through the microarray for measurement purposes.

Preferably the surface structures are formed by etching, lithographic processes, hot embossing, nano-embossing, injection molding or by the Continuous Forming Technology process as described in WO2007/058548.

Preferably the defined areas of the surface structures are functionalized using NH₂ or COOH functional groups.

Preferably one or more single strands of nucleic acid comprising a promoter region or one more single strands of nucleic acid comprising a specific base sequence of interest are attached to the NH₂ or COOH functional groups.

Preferably a linker group is attached to the NH₂ or COOH functional groups.

Preferably the sensory agent is attached to the linker group.

Preferably the sensory agent is attached directly to the NH₂ or COOH functional groups.

Preferably the linker group is a covalent linker group.

Preferably the linker groups are selected from aliphatic compounds, PEG molecules and polymers, proteins or DNA chains.

Alternatively the sensory agent is attached directly to the defined functionalized areas through a linker group.

Preferably the sensory agents are biological recognition groups or binding agents.

Preferably the biological bindings or recognition agents form part of antibody/antigen, DNA/DNA, DNA/protein, protein/protein, protein/receptor, cell/protein and cell/DNA binding partners.

Preferably the three-dimensional microarray includes a plurality of sensory agent groupings, each sensory agent grouping forming a section of the microarray.

Preferably the defined functionalized areas include a plurality of attached sensory agents.

Preferably the three-dimensional microarray is coated between the defined functionalized areas with an inert material.

Preferably, before coating, the microarray is treated with a thiol, protein or PEG material to ensure coating adhesion.

Preferably the inert material is selected from gold or silver or chromium, a polymer or an oil.

Preferably the inert material is a combination of any of gold, silver or chromium.

Preferably the inert material is applied using evaporation, painting, deposition, sputtering, plasma treatment, spray coating, dip coating, or spin coating.

Preferably the thickness of the coating ranges from sub nm to µm to mm measurements.

Preferably the microarray includes a secondary inert coating.

Preferably the secondary inert coating is a thiol compound. Also disclosed is a two-dimensional microarray for use in determining the presence of a target compound(s) of interest within a sample, the microarray including:
a) a base material including a plurality of defined functionalized areas;
b) the plurality of defined functionalized areas including sensory agent(s) capable of attaching to the target compound(s) of interest within the sample.

Preferably a signal entity capable of providing a detectable response is attached to the target compound to be detected in the sample to form a mixed sample, and the sensor response given by the sensory agent(s) in the defined functionalized areas is provided by attachment of the signal entity to the sensory agent(s).

Preferably the detectable sensor response is selected from colour, fluorescence, magnetic or light blocking.

Preferably the detectable sensor response is capable of being read by digital counting, weight measurements, fluorescence, optical, and/or electrical means. Preferably the detectable sensor response results in any one or a combination of quantitative/qualitative, fluorescence, optical or colour metric measurements. Preferably the detectable sensor response is digital.

Preferably the detectable sensor response results in quantitative or qualitative measurements.

Preferably the defined functionalized areas are substantially identical in size and uniformly separated from each other.

Alternatively the defined functionalized areas are randomly placed on the surface of the microarray.

Preferably each defined functionalized area is between about 1 nm and 1000 micron in diameter.

Preferably each defined functionalized area is between about 1 micron and about 20 micron in diameter.

Preferably each defined functionalized area is between about 5 micron and about 15 micron in diameter.

Preferably each defined functionalized area is about 10 micron in diameter.

Preferably each defined functionalized area is about 1 micron in diameter.

Preferably each defined functionalized area is about 500 nm in diameter.

Preferably the tip of the surface structure is the same size or smaller than the size of the signal entity.

Preferably the defined functionalized areas are separated from each other by about 5 nm to about 20 micron spacing.

Preferably the defined functionalized areas are separated from each other by about 1 to about 20 micron spacing.

Preferably the defined functionalized areas are separated from each other by about 5 to about 15 micron spacing.

Preferably the defined functionalized areas are separated from each other by about a 10 micron spacing.

Preferably defined functionalized areas are separated from each other by about a 1 micron spacing.

Preferably the defined functionalized areas are separated from each other by about 5 to about 1000 nm spacing.

Preferably there are between about 250,000 and about 1 billion defined functionalized areas per cm².

Preferably there are about 250,000 defined functionalized areas per cm² at a 10 µm resolution.

Preferably the base material of the two-dimensional microarray is a planar substrate, a spherical substrate or a tubular substrate.

Preferably the two-dimensional microarray is formed from a plastics material, including PMMA, PET or PS, or metals such as aluminium, or ceramics or oxides or silicon or a photoresist or glass.

Preferably the plastics material is selected from PMMA, PET and PS.

Preferably the metal is aluminium.

Preferably the microarray is formed from a polymer substrate.

Preferably the two-dimensional microarray is between about 500 microns and about 2 mm thick.

Preferably a patterned layer is attached to or formed onto the underside of the base material to disperse light across the surface where light is passed through the microarray for measurement purposes.

Preferably the defined functionalized areas are formed by layering an inert material between surface structures of a three-dimensional microarray to form a flat surface and then removing the top layer of the inert material by etching techniques to expose defined areas of the base material of the microarray.

Preferably before layering of the inert material, the microarray is treated with a thiol, protein or PEG material to ensure adhesion of the inert material.

Preferably the inert material is selected from gold or silver or chromium, a polymer or an oil.

Preferably the inert material is a combination of any of gold, silver or chromium.

Preferably the inert material is applied using evaporation, painting, deposition, sputtering, plasma treatment, spray coating, dip coating, or spin coating.

Alternatively the defined functionalized areas are formed by lithographic processes, printing techniques or masking techniques.

Preferably the defined areas are functionalized using NH₂ or COOH functional groups.

Preferably one or more single strands of nucleic acid comprising a promoter region or one more single strands of nucleic acid comprising a specific base sequence of interest are attached to the NH₂ or COOH functional groups.

Preferably a linker group is attached to the NH₂ or COOH functional groups.

Preferably the sensory agent is attached to the linker group.

Preferably the sensory agent is attached directly to the NH₂ or COOH functional groups.

Preferably the linker group is a covalent linker group.

Preferably the linker groups are selected from aliphatic compounds, PEG molecules and polymers, proteins or DNA chains.

Alternatively the sensory agent is attached directly to the defined functionalized areas through a linker group.

Preferably the sensory agents are biological recognition groups or binding agents.

Preferably the biological bindings or recognition agents form part of antibody/antigen, DNA/DNA, DNA/protein, protein/protein, protein/receptor, cell/protein and cell/DNA binding partners.

Preferably the two-dimensional microarray includes a plurality of sensory agent groupings, each sensory agent grouping forming a section of the microarray.

Preferably the defined functionalized areas include a plurality of attached sensory agents.

Preferably the microarray includes a secondary inert coating between the defined functionalized areas.

Preferably the thickness of the secondary inert coating ranges from sub nm to µm to mm measurements.

The microarray described therein wherein
a) the areas on the microarray to be formed into the defined functionalized areas are first covered in a removable blocking material (e.g. wax) and then the areas between the areas covered in a removable blocking material are coated in an inert material;
b) the defined functionalized areas are formed by (i) whole or partial removal of the inert coating to expose the underlying base material to create defined areas and (ii) functionalization of the areas with sensory agent(s); and
c) the inert coating is removed by friction, abrasion, heat, cutting, electrical ablation, microtoming, electropolishing, iron milling, laser or etching techniques.

In a third aspect the invention provides a method for determining whether or not a nucleic acid comprising a specific sequence of bases is present in a sample, the method comprising:
a) in a sample of nucleic acid, where the nucleic acid is double stranded, separating it into single strands;
b) combining the single strands of a nucleic acid with a signal entity conjugate to form a mixed sample;
c) determining whether the nucleic acid comprising a specific sequence of bases is present by running the mixed sample across the surface of a functionalized microarray according to any one of claims 1-11; and
d) counting the number of bound signal entity conjugates.

In a fourth aspect the invention provides a method for determining the extent of methylation in the promoter region of a gene, the said method comprising:
a) in a sample of nucleic acid, where the nucleic acid is double stranded, separating it into single strands;
b) treating the sample of nucleic acid such that non-methylated Cytosine is converted to Uracil;
c) combining the single strands of nucleic acid with a signal entity conjugate to form a mixed sample;
d) determining the presence and/or extent of methylation in the promoter regions by running the mixed sample across the surface of a functionalized microarray according to any one of claims 1-11; and
d) counting the number of bound signal entity conjugates.

Preferably the non-methylated Cytosine bases are converted to Uracil by a chemical conversion using bisulphite.

The methods according to the third and fourth aspects of the invention wherein,
a) preferably the single strands of nucleic acid are functionalized before they are combined with the signal entity conjugate, preferably they are functionalized with a terminal carboxyl or amino functional group;
b) preferably the microarray is a two- or three-dimensional microarray according to the third and fourth aspects of the invention, preferably the defined areas of the microarray are functionalized by the attachment of one or more single strands of nucleic acid;
c) preferably the signal entity conjugate is formed by attaching a signal entity to a complimentary compound which is capable of being bound (directly or indirectly) to the single strands of nucleic acid attached to the defined functionalized areas of a microarray;
d) preferably the mixed sample is prepared using a suitable buffer at a suitable pH, preferably the mixed sample is an aqueous solution;
e) preferably the number of bound signal entity conjugates is ascertained by visual techniques, spectrophotometric techniques, fluorescent techniques, potentiometric or galvanostatic techniques, magnetic light refraction, heat, frequency and digital techniques.
Also disclosed is a method for functionalizing a plurality of defined areas of a microarray for use in the determination of whether or not a molecule or compound is present within a sample and/or whether or not the molecule or compound has been modified in any way, the method comprising attaching the molecule or compound of interest to the defined areas and comprising the following additional steps:
a) forming a signal entity conjugate and attaching the conjugate to molecules or compounds of interest which are attached to the defined functionalized areas;
b) washing off excess signal entity conjugate(s) which have not bound to the molecules or compounds of interest attached to the defined functionalized areas;
c) counting the number of bound signal entity conjugates;
d) releasing the bound signal entity conjugates for use in the fifth and sixth aspects of the present invention, leaving only the molecules or compounds of interest attached to the defined functionalized areas.

Preferably the microarray is a three-dimensional microarray preferably the defined areas of the microarray are functionalized by the attachment of a molecule(s) or compound(s) of interest.

Preferably the signal entity conjugate is formed by attaching a signal entity to a complimentary compound which is capable of being bound (directly or indirectly) to the molecule of compound of interest attached to the defined functionalized areas of a microarray.

Preferably the molecule or compound of interest attached to the defined functionalized area is a single stranded nucleic acid complimentary to a single stranded nucleic acid forming a part of the signal entity conjugate.

Preferably the molecule or compound of interest attached to the defined functionalized area is a peptide, antibody, or microorganism (for example, virus particles or bacteria).

Preferably the excess signal entity conjugate(s) are removed from the surface of the microarray by washing with a carrier solution.

Preferably the carrier solution is a buffer.

Preferably the number of bound signal entity conjugates is ascertained by visual techniques, spectrophotometric techniques, fluorescent techniques, potentiometric or galvanostatic techniques, magnetic light refraction, heat, frequency and digital techniques.

Preferably the bound signal entity conjugates are released from the defined functionalized areas in response to a change in their environment, including and not limited to a change in the pH of the aqueous solution.

Preferably the release of the signal entity conjugates is reversible.

Preferably this reversibility is achieved by changing the pH of the aqueous solution.

Preferably this reversibility allows the microarrays to be stored and used a number of times.

Preferably the microarrays are stored in a fridge at about 4°C.

Also disclosed is a method for functionalizing a plurality of defined areas of a microarray for use in the determination of whether or not a nucleic acid comprising a specific sequence of bases is present within a sample and/or in the determination of the presence and/or extent of methylation within the promoter region of a single strand of nucleic acid, the method comprising attaching a single strand of a nucleic acid to the defined areas according to the third or fourth aspects of the present invention and comprising the following additional steps:
a) forming a signal entity conjugate and attaching the conjugate to a nucleic acid which is attached to the defined functionalized areas;
b) washing off excess signal entity conjugate(s) which have not bound to the nucleic acid attached to the defined functionalized areas;
c) counting the number of bound signal entity conjugates;
d) releasing the bound signal entity conjugates for use in the third and fourth aspects of the present invention, leaving only the nucleic acid attached to the defined functionalized areas

Preferably the single strand of a nucleic acid is attached to the defined areas through a carboxyl or amino group using standard techniques including and not limited to DCC coupling.

Said method may comprise attaching a compound to the defined areas of a microarray and may comprise the following additional steps:
a) providing a complimentary compound and attaching the complimentary compound to the compound(s) attached to the defined areas;
b) attaching a signal entity to the complimentary compound to form a signal entity conjugate on the defined areas;
c) counting the number of bound signal entity conjugates;
d) releasing the signal entity conjugates to leave only the compounds attached to the defined areas.

Preferably the signal entity conjugates are formed by washing signal entities over the surface structures of the microarray as an aqueous solution.

Preferably the signal entity employed is as described in the first and second aspects of the present invention.

In a preferred embodiment the method may comprise attaching a single strand of a nucleic acid to the defined areas of a microarray and may comprise the following additional steps:
a) synthesizing a complimentary strand of the nucleic acid and attaching the complimentary strands to the bound nucleic acid;
b) attaching a signal entity to the complimentary strands to form a signal entity conjugate on the defined areas of the microarray;
c) counting the number of bound signal entity conjugates;
d) releasing the signal entity conjugates to leave only the single strands of nucleic acid attached to the surface structures.

Preferably the complimentary strands are synthesized using a nucleic acid synthesizer and functionalized with a terminal carboxyl or amino group and are bound to single strands of a nucleic acid through complimentary interactions between carboxyl and amino groups of the nucleic acid strands involved.

In a ninth aspect the invention provides a method for determining the potential and/or the real effect of environment, diet, or medication on nucleic acid, the method including the use of the method according to the sixth aspect of the invention to determine the extent of methylation in the promoter region of the nucleic acid.

### Drawing Description

- Figure 1:: shows, in diagrammatic form, the preparation of a Three-Dimensional MicroCone Array Substrate;
- Figure 2:: shows, in diagrammatic form, stylised depictions of surface structures of the three-dimensional microarrays;
- Figure 3:: shows a side view of a diagrammatic expression of the creation of a two-dimensional microarray from a three-dimensional microarray;
- Figure 4:: shows a top view of a diagrammatic expression of the creation of a twodimensional microarray from a three-dimensional microarray;
- Figure 5:: shows, in diagrammatic form, the preparation of a Digital BiosensingSingle "Sandwich" Assay;
- Figure 6:: shows, in diagrammatic form, the preparation of a Digital BiosensingMultiple "Sandwich" Assay;
- Figure 7:: shows, in diagrammatic form, an alternative method for functionalizing the microarrays as described in detail in one embodiment of the invention;
- Figure 8:: shows, in diagrammatic form, how the microarrays are functionalized as described in detail in one embodiment of the invention;
- Figure 9:: shows, in diagrammatic form, how the microarrays are employed as detectors when functionalized as depicted in Figure 5;
- Figure 10:: shows, in diagrammatic form, how the microarrays are employed as detectors when functionalized as depicted in Figure 7;
- Figure 11:: shows digital images of functional three-dimensional microcone arrays.

### Detailed Description

The invention concerns the development of three-dimensional microarrays for use in detection/sensing applications with high sensitivity and selectivity. In particular the invention relates generally to the detection of compound(s) in a sample and also to the determination of whether a nucleic acid comprising a specific sequence of bases is present within a sample, quantifying the number of the specific nucleic acid base sequences within a sample, determining the presence and/or extent of methylation in the promoter region for a particular gene, and the effects of various factors, including environment, diet or medications on nucleic acid methylation.

The invention in a general sense provides a method for determining the presence of a target compound(s) of interest within a sample, the method including the steps of:
a) Providing the three-dimensional microarray of any one of claims 1-11; and
b) Passing at least part of the sample over the array; and
c) Determining the presence of a target compound(s) of interest by detection of a detectable response to the attachment of the target compound(s) to the sensory agents(s).

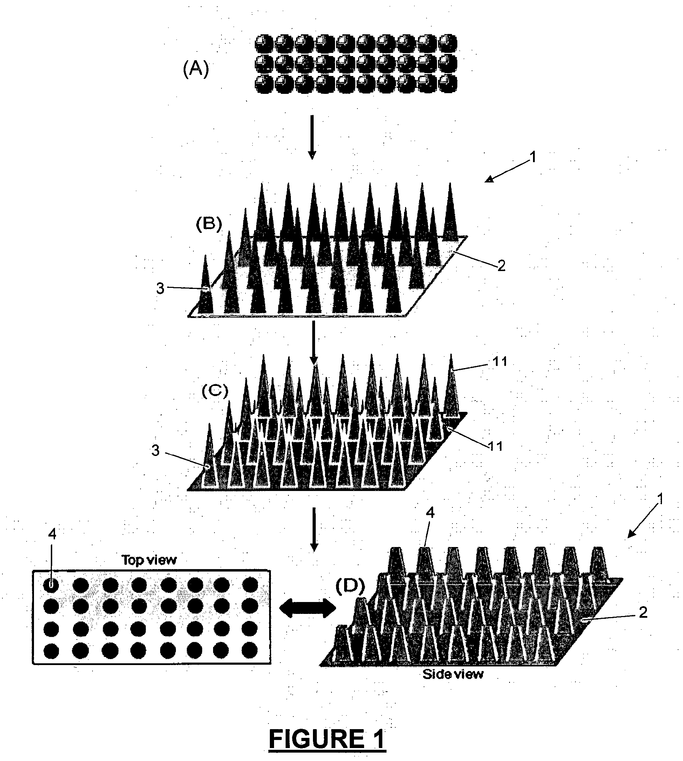

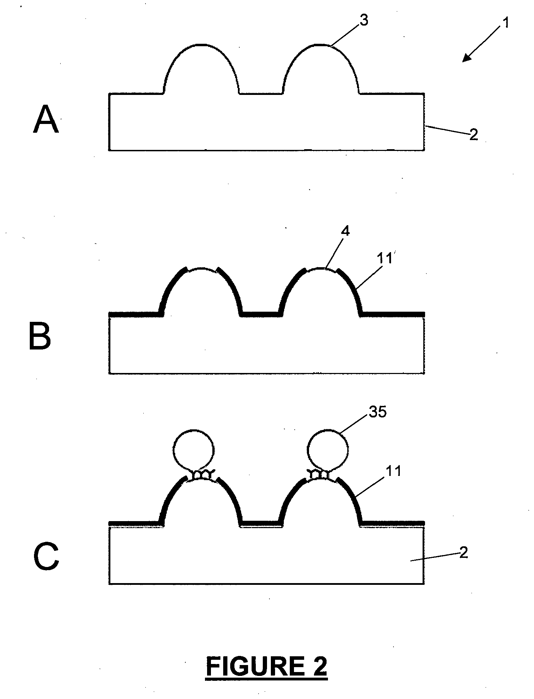

The invention also provides a three-dimensional microarray for use in determining the presence of a target compound(s) of interest within a sample, the microarray including:
a) a base material including a plurality of surface structures forming part of and protruding from the base material;
b) the plurality of surface structures including sensory agent(s) capable of attachment to the target compound(s) of interest within the sample, the sensory agent(s) being included on defined functionalized areas on the tops of each surface structure.;
and wherein the microarray is coated between the defined functionalized areas with an inert material to minimize non-specific binding. With reference to Figures 1 and 2, the base material of the microarrays 1 according to the present invention, consists of a flat base 2 ranging in thickness from about 500 microns to about 2 millimeters. For three-dimensional microarrays defined functionalized areas take the form of surface structures 3 (e.g. "cones" or "ridges") which protrude (Figure 1, (B), Figure 2, (A)) from the flat base. For two-dimensional microarrays the defined functionalized areas are flat sensor sites and therefore do not protrude from the base. The flat base of the two-dimensional microarray can be a planar substrate, a spherical substrate or a tubular substrate. The tops of the surface structures 4 and the flat sensor sites form defined functionalized areas on the microarrays and include sensory agent(s) capable of attaching to the target compound(s) of interest within the sample. Attachment of the sensory agent(s) to the target compound(s) results in, or can be made to result in, a detectable response, thus those defined areas can be "functionalized" to give sensor responses. Preferably these defined areas 4 are millimeter to nanometer sized areas which are substantially identical and uniformly separated from each other. Alternatively, these defined functionalized areas are millimeter to nanometer sized areas which are randomly ordered on the surface of the microarray.

In a preferred embodiment, the microarrays are three-dimensional microarrays wherein uniformly spaced cones or ridges form part of the base material to allow for more accuracy in the end application. The cones or ridges typically range in size from about 100 nm to about 10 mm in diameter at the base and about 1 nm to about 1000 micron in diameter at the tip. Alternative tip diameters are available, as would be known to a skilled person in the art, and include ranges between about 1 micron to about 20 micron and about 5 micron to about 15 micron. The tips may also be as small as about 10 micron, about 1 micron or about 500 nm in diameter. The cones or ridges are tightly packed in a defined pattern. For example cones with a 1 micron tip size which are separated by a 1 micron spacing from all other tips will produce an array of 25 million tips per cm², while a 500 nm tip separated by a 500 nm spacing from all others will produce an array of 100 million tips per cm². The cones or ridges may be separated from each other by about 5 nm to about 20 micron. Preferably, the cones or ridges are separated from each other by a spacing of about 1 micron to about 20 micron. Alternative spacings of about 5 micron to about 15 micron or about 10 micron, or about 1 micron, or about 5 nm to about 1000 nm are also available. Preferably there is anywhere from about 250,000 functionalized tips at a 10 µm resolution to about 100 million functionalized tips per cm². The defined functionalized areas (indicated as 4 in Figures 1 and 2) will be present on the tips of the cones or ridges, thus the sizes of, and separation of, these areas and the resultant numbers of functionalized defined areas reflects those separations and numbers. Consequently, the user knows exactly how many functionalized tips are on the surface of the microarray. The overall size of the microarrays can be altered according to the end user's needs. This is a major advantage of the present invention. Typically, the size area of the microarrays range from as small as about 10 mm² to about 150 mm². Size areas outside this range can be achieved. As seen at Figure 2C, following functionalization of the defined areas, the presence of a target analyte in the sample can also be detected by attachment of a microbead conjugate 35 to the target analyte, said microbead conjugate 35 being capable of attaching to the sensory agent(s) to give a sensor response. This is discussed in more detail below.

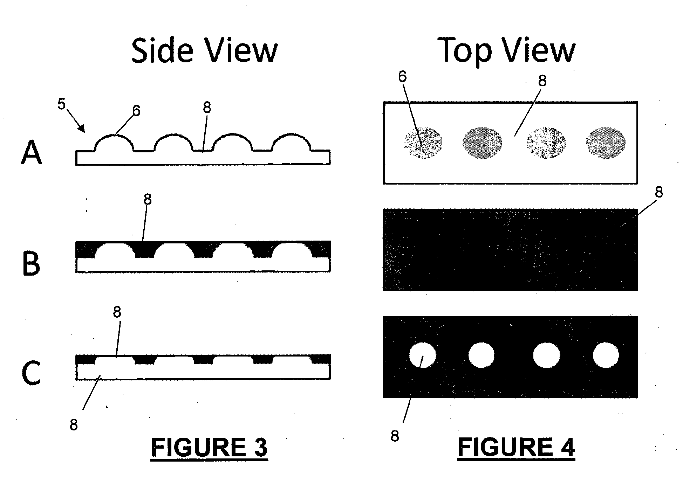

In another embodiment the microarrays are two-dimensional microarrays (not shown in Figures 1 and 2 - best seen in Figure 4) wherein the flat defined functionalized areas range in size from about 1 nm to about 1000 micro in diameter. Preferably, the flat defined functionalized areas range in size from about 1 micron to about 20 micron, or about 5 micron to about 15 micron. The flat defined functionalized areas may also be as small as about 10 micron, about 1 micron or about 500 nm in diameter. The defined functionalized areas are tightly packed in a defined pattern. For example defined functionalized areas with a 1 micro diameter which are separated by a 1 micro spacing will produce an array of 25 million defined functionalized areas per cm². Ideally the defined functionalized areas are separated from each other by a spacing of about 1 micron to about 20 micron. Alternative spacings of about 5 micron to about 15 micron or about 10 micron, or about 1 micron, or about 5 nm to about 1000 nm are also available. Preferably there is anywhere from about 250,000 defined functionalized areas at a 10 µm resolution to about 100 million defined functionalized areas per cm². As a consequence of the uniform spacing, the user knows exactly how many defined functionalized areas are on the surface of the microarray. As with the three-dimensional microarray the size of the two-dimensional microarray can be altered according to the users end needs.

It is important to note that where the defined functionalized areas are randomly ordered on the surface of the microarray, exact quantification of the number of defined functionalized areas on the surface of the microarray is not known until after the user has digitally scanned the surface of the microarray. Further, it is possible that not all of the defined areas will be functionalized, even though the intention may be to do so. Reference to functionalization should therefore be seen as reference to functionalization of substantially all of the defined areas to reflect reality, unless the context is clearly otherwise.

The base material of the microarrays can be formed from a plastics material, including PMMA, PET and PS or metals such as aluminium or ceramics or oxides or silicon or a photoresist (Figure, 1(A)). Unlike the three-dimensional microarray, the two-dimensional microarrays can also be made out of materials such as glass. Preferably the base material of the microarray is made from polymer substrates for cost and processing advantages. Any process capable of manufacturing surface structures can be employed to manufacture the base material of the three-dimensional microarrays. For example, etching techniques, lithographs processes, Continuous Forming Technology, hot embossing, nano-embossing and injection molding can be employed. A preferred process for forming the base material of the three-dimensional microarrays is Continuous Forming Technology as described in WO2007/058548. Where this process is employed the base material can be mass produced easily and at low cost.

The two-dimensional microarrays can be formed by layering an inert material between individual structures of a three-dimensional microarray to form a flat surface. The inert material is preferably gold, silver or chromium. Alternatively, the inert material may be a polymer or an oil. The inert material is introduced using any one of a number of different coating methods, including and not limited to, evaporation, painting, deposition, sputtering, plasma treatment, spray coating, dip coating and spin coating. Etching techniques can then be employed to remove the top of inert material to create a flat surface on which defined areas of the base material are exposed and are therefore available to act as defined functionalized areas. This method of forming two-dimensional microarrays is particularly suitable where the height of the structures of a three-dimensional microarray is the same or less than the thickness of the coating layer (sub nm to µm to mm). Removal of the top of the coating layer will then result in an essentially flat two-dimensional microarray having defined areas that can be functionalized as desired. Figures 3 and 4 show a diagrammatic expression (side view and top view, respectively), of the creation of a two-dimensional microarray from a three-dimensional microarray. As can be seen in Figure 3, the three dimensional initial structure 5 has a plurality of surface structures 6 on a base 7. The areas 8 between the surface structures 6 are filled in with a coating layer 9 as seen in Figure 3B. As can also be seen in Figure 3B, the coating layer 9 also covers the top of the surface structures 6. To form the defined areas to be functionalized, the top of the coating layer 9 is removed (Figure 3C) as are the tops of the surface structures 6. This leaves defined areas 10 on the flat surface that can be functionalized as desired. The microarray is now two-dimensional. With reference to Figure 4, the top view of the microarray formation of Figure 3 is shown. Figure 4B shows how the surface structures 6 are covered with the coating layer 9 (Figure 4B) which is then partially removed to leave defined areas 10 (Figure 4C) that can be functionalized.

Alternative methods of forming two-dimensional microarrays include, and are not limited to, lithographic, printing or masking techniques. However, alternative techniques may be used. Where masking techniques are employed, a masking element is applied to the flat base in a set pattern and both are then coated with an inert material. The masking element is then removed to leave behind a substrate with uncoated areas. These uncoated areas are then available to act as defined functionalized areas.

The surface structures on the three-dimensional microarray (e.g. cones or ridges) or the flat defined areas on the two-dimensional microarray forming part of the base material creates a substrate on which a defined functionalized area can be created. This area is formed through functionalization of the tops (or tips) of the cones or ridges, or functionalization of the flat areas.

First, a functional group is attached to the tips or flat sensor sites. Functional groups such as -NH₂ or -COOH are typically employed. However, many other functional groups can be used, including the likes of aldehydes and thiols. Preferably a linker group is then bound to the -NH₂ or -COOH functional groups. Many different linker groups can be employed including, and not limited to, long or short aliphatic chains, PEG molecules and polymers, protein chains or DNA chains. Various techniques can be employed to introduce the linker groups including, and not limited to, plasma treatment and wet chemistry techniques.

Next a sensory agent is immobilised onto (attached to) the linker group. Alternatively the sensory agent may be attached to the -NH₂ or -COOH functional group without a linker, but the use of linkers is a preferred option. Sensory agents are selected from biological recognition groups, or binding agents and include the likes of antibody/antigen, DNA/DNA, DNA/protein, protein/protein, protein/receptor, cell/protein and cell/DNA biological pairings. These are selected according to the nature of the target compound(s)/molecule(s) the user wishes to detect. Thus, the sensory agent(s) must be capable of acting as a biological recognition group or binding agent (i.e. attaching) to the target compound(s)/molecules(s) of interest.

In the sequence just described covalent linking is employed between each of the layers, namely the base material, functional groups, linkers and the sensory agent, to form the attachments. Covalent linking is preferred, but is not necessary. For example, and where appropriate, non-covalent linking techniques, such as electrostatic absorption and charge based absorption, can be employed.

Where steric hindrance poses a problem for the attachment of the sensory agent due to its size, an additional linker may be inserted between the defined functionalized area surface and the sensory agent. The sensory agents can also be bound directly to the cone, ridge tips or flat sites, provided a linker, preferably covalent as stated above, is incorporated into the tips or flat sensor sites of the base material.

It is the addition of a sensory agent which allows the microarrays to act as digital sensors with quantitative/qualitative, fluorescence, optical or colour metric measurements resulting. This is because, when a target analyte recognises the sensory agent, it binds itself to, or becomes attached to, the sensory agent and thus becomes attached to the microarray. This attachment of the analyte to the sensory agent on the microarray results in, or can be made to result in, a sensor response and allows the number of attached analytes to be 'counted'. Thus, the user canaccurately ascertain the proportion of target analytes in their sample. Quantitative/qualitative measurements are preferred in terms of sensor response, as these can be accurately interpreted by digital means.

However, many other techniques can be also employed for ascertaining the extent of target analyte attachment including and not limited to weight measurements, fluorescence, optical, colour metric and/or electrical (potentiometric or galvanostatic) techniques. Where digital measurements are preferred, these can be made by commercial MicroScanners, Microscopes and/or digital imaging, with or without the need to pass light through the sample. Alternative methods could be employed, as would be known to a person skilled in the art. Where light is passed through the sample, a patterned layer attached to or formed onto the underside of the base material (by etching techniques or the like as discussed earlier) may be required to diffuse light across the surface. Employment of different wavelengths of light may allow the user to detect different target analytes within a sample and/or the occurrence of a reaction and/or information about the bound target analyte, e.g. the recognition of the cell type or viability.

In one embodiment, the present description provides a potentially useful screening method for distinguishing target analytes that may be present in a sample based on their size and/or shape. For example, target analytes such as bacteria, may exhibit a particular shape and/or size when bound to the sensory agents on the defined functionalized areas of a microarray according to the invention. The shape and/or size can be determined by passing light through the microarray and assessing the number of defined functionalized areas which are blocked by the target analyte. This will directly indicate size but indirectly indicate shape as the blocked defined areas will effectively form a shape. It is likely that the target analyte, such as bacteria, will usually bind to sensory agents on the defined functionalized areas of the microarray in the same manner. Therefore, target analytes of the same type could potentially exhibit the same rough shape and/or size when bound to sensory agents on the defined functionalized areas of the microarrays according to the present invention. It is in this way that the present invention provides a screening method as the user can potentially create a key based on different shapes and/or sizes of different target analytes. It is preferable that the sample employed for such a screening application is dilute as this will better enable the user to distinguish target analytes based on their shape and/or size.

Where the target analyte cannot be observed directly signal entities with dimensions of nanometers to millimeters and specific properties depending on the response required (for example, colour, fluorescence, magnetic, heat, electrical or light blocking) may be attached to the remaining sensory agents by simple physical adsorption techniques or by covalent linkage through different coupling chemistry techniques. Conversely, signal entities can be attached to the bound target analytes by similar means.

Preferably, the signal entities employed are the same size or larger than the tip of the surface structure or the defined flat areas (of a two-dimensional microarray). The signal entities may be of any shape and may be of such a size that they cover a number of individual defined areas on the microarray surface. Preferably the signal entities take the form of particles such as coloured, fluorescence, magnetic or light blocking microbeads. Where coloured, fluorescent or light blocking particles are employed, the number of particles bound to the microarray can be counted. Where different shaped signal entities and/or different sized signal entities are employed, the response is visual, i.e. the user is able to detect the presence of different target analytes based on the differentiation in size and/or shape of the signal entities. Alternative sensor responses include qualitative or quantitative responses such as weight measurements, fluorescent responses, spectrophotometric responses, potentiometric or galvanostatic responses, magnetic light refraction, heat and frequency responses. The signal entities can also be used to form signal entity conjugates, which are discussed later herein in more detail.

In another embodiment, the present description provides a means of determining the nature of the target analyte present. For example, it is well known that a single type of bacteria can have a number of strains. The user may functionalize the microarray with a sensory agent that binds to a number of strains, such that when a functionalized microarray is exposed to a sample, the user will be able to determine in general terms if a certain bacteria is present within the sample. The user may then expose the attached bacteria to particles (for example, signal entities) which have been functionalized to discriminate between the particular strains. This could be done by washing the microarray having attached target analytes/bacteria with a composition including a signal entity that will specifically bind to a certain strain. Where targeted strains are present, the particles will attach and give a sensor response.

The choice of sensory agent allows the user to manipulate the defined functionalized areas to sense one or a number of target analytes. For example, different sensory agents can be attached to the defined functionalized areas such that different sections of the microarray hold different sensory agents. Furthermore, the close packing of the defined functionalized areas, and the correspondingly high number of defined functionalized areas per cm² as described above, results in a microarray of high sensitivity. For example and as per above, where one sensory agent is attached to each defined functionalized area on the tips of the surface structure of a three-dimensional microarray, a 1 micron tip separated from all others by a 1 micron spacing will produce an array of 25 million functionalized tips per cm². Likewise, where the tip diameter is 500 nm, the array will contain 1 billion defined functionalized areas (i.e. functionalized tips) per cm². The high number of defined functionalized areas enables high throughput screening and/or analysis of target analytes. Addition of one sensory agent per defined functionalized areas is preferable as this gives rise to a highly sensitive sensor surface and allows for direct digital counting of the target compounds/analytes that attaché to the agent. Increased sensitivity is achieved by ensuring the defined functionalized areas are the same size, or smaller, than the target analyte(s) or the attached particle, as defined above. Where appropriate, a larger cone, ridge tip or flat sensor site, and hence a larger defined functionalized area, can be employed, allowing for the addition of multiple sensory agents to the defined functionalized areas. This results in the formation of a sensor which is similar to a strip test. For example, if the cone tip size (i.e. defined functionalized area) of a three-dimensional microarray is 200 micron and the sensory agent is only 1 micron in size, a microarray comprising millions of individual and identical strip sensors can be formed. While individual counting of attached compounds/analytes may no longer be possible, the user remains able to detect the presence of targets in their sample and may potentially be able to carry out real time detection experiments. The high sensitivity of the microarray sensor surface also potentially allows for single molecule detection, particularly where the defined functionalized areas can be made smaller than the µm level.

Further increased sensitivity of a three-dimensional microarray is achieved by coating the base material of the microarray with an inert material to minimize non-specific binding, allowing for more accurate measurements such as digital counting. This can best be seen with reference to Figures 1 and 2. Figure 1B shows the surface structures 3 and the base 2 in an initial uncoated form. Figure 1C then shows the surface of the microarray, as a whole, having been coated with a layer 11 of a suitable inert material. Figure 1D then shows the creation of defined areas 4 for functionalization that have been created by removal of the coating layer 11 from the top of the surface structures 3 as well as a part of the surface structures 3 themselves. The side view of Figure 1D shows how this creates a pattern of defined areas 4. This can also be seen depicted in Figure 2. To achieve this, the inert material is introduced to the base material surface using any one of a number of different coating methods, including and not limited to, evaporation, painting, deposition, sputtering, plasma treatment, spray coating, dip coating and spin coating.

As indicated earlier, where the areas between the individual structures of a microarray are completely filled with an inert material, a two-dimensional microarray may be formed.

The inert material itself can be almost anything that results in a non-reactive surface, including metals such as gold or silver or chromium, or polymers, paints and oils. The inert material can also consist of a combination of metals used together. The thickness of the inert coating can range from sub nm to µm to mm depending on the use to which the microarray is to be put. Where adhesion of the inert material is a problem, the base material can be treated, prior to coating the inert material, with the likes of thiol-based compounds, proteins or PEG molecules/polymers. In the case of a gold coating and where non specific binding remains a problem, a secondary coating of the likes of a thiol can be applied to further reduce the non-specific binding.

Once the base material is coated, it is preferably treated to remove small specific areas of coating, allowing for functionalization of defined areas of the microarray (as described above and represented in Figure 2, (C)) and thus creating its corresponding sensor capabilities.

Preferably, in the case of a three-dimensional microarray, it is the tip of the cone or ridge structure (or other like structure) from which any coating is removed (Figure 1(D), Figure 2, (B)). In the case of a two-dimensional microarray, etching techniques may be used to form the flat defined areas. Preferably the coating is completely removed from these areas to expose the underlying base material. Alternatively, the coating may be only partially removed from these areas such that a thin coating of the inert material remains. Surprisingly, the inventors have found that the presence of a very thin coating does not substantially inhibit functionalization of the defined areas.

Various methods can be employed to remove the coating from the tips of the cones or the flat defined areas (i.e. potential sensor sites). However, the method employed should preferably be able to remove an accurate size and depth of inert coating from the tips. For this reason friction, abrasion, heat, cutting, electrical ablation, microtoming, electropolishing, iron milling, laser and etching techniques are commonly employed where the microarray is a three-dimensional microarray.

Conversely, masking techniques can also be employed to ensure any inert coating only ends up on the sides and valleys (and not the tips) of the base material of the three-dimensional microarray. Using this technique, the cones or ridges could be masked or protected by dipping the base material into a wax solution upside down or the die surface used to create the surface structures (which may have an aperture at the tip) could be used as a mask. The masking compound or die surface is then removed if it does not have an aperture at the tip to allow for functionalization. Masking or etching techniques or lithographic or printing techniques can be employed to remove the coating from the defined areas of a two-dimensional microarray.

Gold is the preferred inert coating material. However, where this is employed a secondary thiol coating should preferably be used to further minimise non-specific binding. Evaporation techniques provide an ideal method of applying the gold coating to the base material.

Gold-covered three-dimensional microarray sensors can be employed for digital biosensing of "competition" or "inhibition" assays, particularly where small molecule compounds (or analytes) such as antibiotics, steroid hormones, drug residues and the likes of small protein or DNA samples are the desired target compounds.

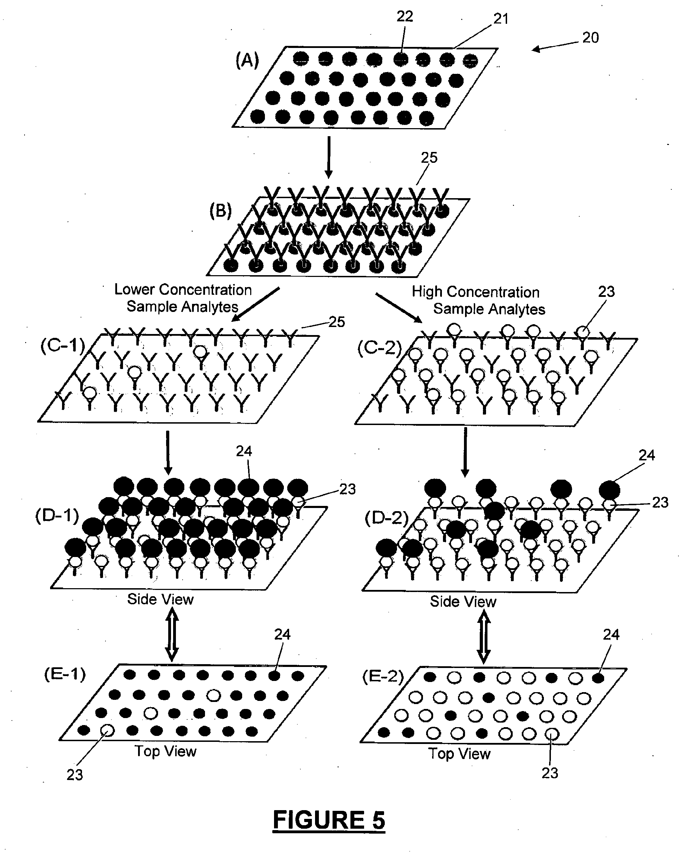

For example, and with reference to Figure 5, gold-covered three-dimensional microarrays 20 have been employed in a "competition assay" of antibody/milk antibiotics or Ab/Ag pairs. After removal of the gold coating 21 from the cone tips 22 (Figure 5, (A)) they are functionalized with an *anti*-antibiotics antibody Y, 25, in a borate buffer (pH 8.5) which is immobilised onto the surface of the microarray (Figure 5, (B)). The microarray is then left overnight before being exposed to a 2% OVA solution in PBS to prevent or eliminate non-specific binding that has taken place on the remaining gold-coated surface. The microarray 20 (Figure 5(B)) is now ready to use as a sensor.

A solution of milk containing a known milk protein 23 is then passed over the microarray. This known milk protein is the target analyte which antibody Y, 25, should bind to. At low concentrations of the protein, few bindings are observed (Figure 5, (C-1)). Conversely, at high concentrations a number of bindings are observed (Figure 5, (C-2)).

The microarray of Figure 5, (C-1) and (C-2) is then exposed to coloured microbead/anti-protein antibody conjugates 24, which bind, or attach, to the surface-bound milk protein 23 via different epitope (Figure 5, (D-1) and (D-2)). The microbead 24 acts as a signal entity that provides a detectable signal. Where there are few analytes 23 bound, attachment of a number of microbead/antibody conjugates 24 is observed and vice versa. Digital assay of the microarray is then carried out (Figure 5, (E-1) and (E-2)). This involves counting either the number of coloured microbead/antibody conjugates or the number of empty cone tips directly. The concentration of the milk protein is proportional to the number of coloured microbeads (sandwich assay), but inversely proportional to the number of empty cone tips. Thus, a high proportion of coloured beads will indicate a high concentration of target analyte, and vice versa.

Where an "inhibition" assay format is employed, it is the small analyte molecule (such as a milk antibiotic as the target molecule) which is immobilised onto the surface of the microarray. Sensor surfaces in inhibition assay formats can be regenerated many times for multiple measurements, while competition assays are generally used only once as disposable sensors.

The above methodology can be repeated in standard form, whereby the functionalized microarray, is exposed to a solution containing a target analyte. These bind to the sensory agents on recognition and when light is passed through the microarray, the defined functionalized areas which have sensory agents bound to target analytes will effectively be blocked by the target analyte allowing for digital counting of the "darkened" areas.

Gold-covered three-dimensional microarray sensors can also be employed in multiple "sandwich" assay digital biosensing formats where the user wishes to detect large target compounds or analytes such as proteins, virus cells, and cells. Such analytes typically have multiple attachment sites (termed epidopes) for sensory agents.

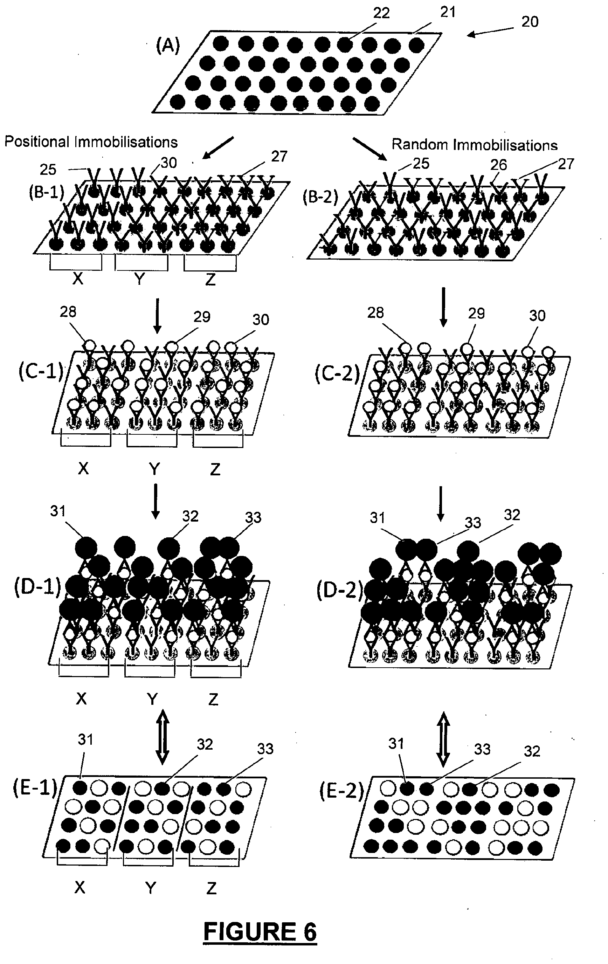

For example, and with reference to Figure 6, three different antibodies 25, 26, 27 (in areas X, Y and Z) are either immobilised in a set position onto the gold-free and functionalized cone tip 22 surfaces of the microarray 20 (Figure 6, (B-1)), or are immobilised randomly onto the cone tip 22 surfaces (Figure 6, (B-2)).

The microarrays 20 are then exposed to 2 % OVA in PBS to block any non-specific binding on the gold surface 21. Three different large analytes 28, 29, 30 are then exposed to the sensor surface. These bind to their respective antibodies 25, 26, 27 (Figure 6, (C-1) and (C-2)).

Next, three differently coloured microbead/antibody conjugates 31, 32, 33 bind to the three different large analytes 28, 29, 30 separately (sandwich assay) to form multicoloured arrays on the surface of the sensor (Figure 6, (D-1) and (D-2)).

The sample concentrations of the three analytes 28, 29, 30 can then be ascertained by counting each of the three different coloured microbeads 31, 32, 33 separately or by carrying out digital, weight, fluorescence or electronic measurements. Concentrations of the analytes 28, 29, 30 are directly proportional to the number of coloured microbeads 31, 32, 33 on the sensor surface (Figure 6, (E-1) and (E-2)), such that a sandwich assay format is generally more sensitive than a competition or inhibition assay format. Such multiple analyses format can also be applied to "competition" or "inhibition" assays for digital biosensing small molecular analytes.

In a preferred embodiment of the present invention two- and three-dimensional microarrays can be employed for determining whether or not molecules or compounds of interest are present in a sample and/or have been modified in any way. The molecules or compounds may be of any biological or chemical nature and include, but are not limited to, nucleic acids, peptides or proteins, micro-organisms (including but not limited to prions, viruses, bacteria), antibodies or any type of small chemical molecule, for example. Examples of the types of modification for which the invention could be used to detect include, but are not limited to, structural changes, substitutions, post transcriptional and post translational modifications (including glycosylation), and methylation of nucleic acids.

The invention may be of use for a number of applications, including diagnostic and forensic applications, for example to identify the presence or absence of a specific nucleic acid sequence (including mutations in a nucleic acid sequence which may signal disease), or a methylation pattern that may be associated with disease.

The description hereinafter focuses on the analysis of a target compound being nucleic acid molecules. However, it should be appreciated that the general methodology described is applicable to other molecules or other target compounds as mentioned above. Therefore, a skilled person will recognise that the invention has many uses.

To determine whether or not a molecule or compound is present within a sample and/or whether the molecule or compound has been modified in any way, the sample of interest is first combined with a signal entity conjugate (the formation of which is discussed in more depth below) to form a mixed sample. The mixed sample is then run across the surface of a two- or three-dimensional microarray and the number of bound signal entity conjugates are counted. Preferably, the mixed sample is an aqueous solution and is prepared using a suitable buffer, for example PBS, at a suitable pH. Preferably the pH is between about 4.0 to about 9.0, more preferably between about 7.0 to about 7.5. Preferably, the signal entity conjugates only bind to the molecule or compound of interest within the mixed sample to form signal entity conjugate complexes.

Therefore the present invention provides a method of determining whether or not one or more nucleic acids (for example, a DNA or RNA strand), comprising a specific sequence of bases is present in a sample. Likewise the invention provides a method for determining the presence and/or extent of methylation of a nucleic acid, preferably methylation within or around the promoter of a gene (herein referred to as the promoter region). A gene comprising a methylated promoter region is unavailable for transcription purposes.

The method for determining whether or not one or more nucleic acids comprising a specific sequence of bases is present in a sample the following preparatory steps must be carried out. First, a sample of nucleic acid is collected. The nucleic acid may be obtained from any appropriate biological material, including but not restricted to a tissue sample, fluid sample or an oral swab. Where the nucleic acid is double stranded it is separated into single strands using known techniques. Second, the single strands are combined with a signal entity conjugate (the formation of which is described below) to form an aqueous mixed sample. The mixed sample is run across the surface of a single (if the assay is for a single base sequence) or multi- (if the assay is for more than one base sequence) functionalized three-dimensional microarray described above.

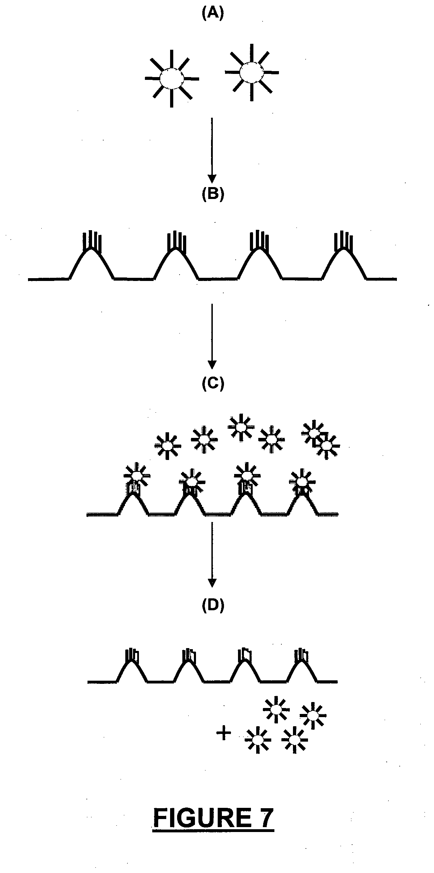

The method for determining the presence and/or extent of methylation of a nucleic acid also forms an aspect of the present invention and comprises the following preparatory steps. First, a sample of nucleic acid is collected from an appropriate biological material as described above for the first preferred embodiment of the first aspect of the invention. Where the nucleic acid is double stranded it is separated into single strands using known techniques. Second, the promoter regions within the single strands of nucleic acid are treated such that all non-methylated Cytosine bases present are converted to Uracil. Preferably the conversion of non-methylated Cytosine to Uracil is achieved by a chemical conversion whereby the non-methylated Cytosine bases are sulfonated and deaminated upon reaction with bisulphite (Nucleic Acids Research, (1996) Vol 24, No. 24, pp 5064-5066). It is known that bisulphite will only react with non-methylated Cytosine bases. Therefore any methylated Cytosine bases present within the promoter region remain unchanged. Third, the treated single strands of nucleic acid are combined with a signal entity conjugate (the formation of which is described below) to form an aqueous mixed sample. Following completion of these steps, determination of whether the Cytosine bases have been converted and have therefore not been methylated is carried out using a single (if the assay is for a single promoter type) or multi- (if the assay is for more than one promoter type) functionalized three-dimensional microarray described above. With reference to Figures 7 to 10 the following key can be used:
Promoter region or base sequence of interest (P1):
   Signal entity: 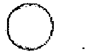
   Signal entity conjugate: 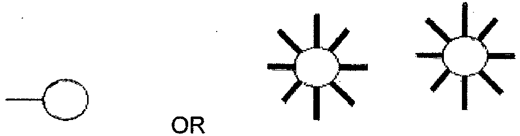
   Signal entity conjugate/nucleic acid complex: 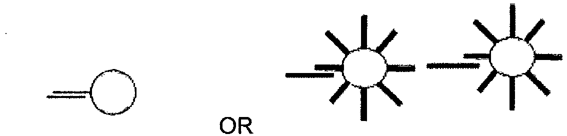
   Nucleic acid containing P1:
      For the purpose of the present invention as it relates to the detection of nucleic acids (and with reference to Figure 7), the defined areas of three-dimensional microarrays, respectively, can be functionalized according to the following methodology (preferably the remainder of the surface of the microarray, other than the defined areas, will have been coated with an inert material):
         a) one or more single strands of a nucleic acid comprising a promoter region or specific base sequence of interest is bound to the defined areas (Figure 7, (B));
         b) a signal entity conjugate is formed and then washed over the defined areas of the microarray allowing for attachment of the conjugate to the bound nucleic acid (Figure 7, (C));
         c) excess signal entity conjugates which have not bound to the nucleic acid are washed off allowing the user to ascertain or count the number of signal entity conjugates bound to the defined areas of the microarray; and
         d) the bound signal entity conjugates are released leaving only the nucleic acid(s) of step (a) bound to the defined areas of the microarray (Figure 7, (D);
         e) The areas are now defined functionalized areas including a known amount and type of sensory agent (the nucleic acid).

A plurality of nucleic acids comprising promoter regions or other base sequences of interest can be bound to the defined areas through carboxyl or amino groups using standard techniques. Dicyclohexylcarbodiimide (DCC) coupling provides an example of a suitable technique for this purpose. Other coupling techniques may be used, including the use of linker molecules, as described earlier. Likewise, each defined area may be functionalized by only one nucleic acid comprising a promoter region or other nucleic acid sequence of interest.

Broad methodology for functionalizing the microarray and forming a signal entity conjugate where molecules or compounds other than nucleic acid are of interest are broadly described above in relation to the detection of target analytes. Skilled persons will readily appreciate appropriate compounds and conditions for attaching such compounds or molecules to the flat sensor sites, surface structures and signal entities, having regard to the nature of the compounds of interest.

The signal entity conjugate is preferably formed by attaching a signal entity to a synthesized complimentary copy of the nucleic acid comprising a promoter region or other base sequence of interest. The complimentary copies of nucleic acids are preferably synthesized using a nucleic acid synthesizer, and are functionalized with a terminal functional group to which the signal entity attaches. Alternatively, the complimentary copies are obtained using recombinant techniques. Examples of suitable terminal functional groups include carboxyl or amino groups. Dicyclohexylcarbodiimide (DCC) coupling provides an example of a suitable standard technique for coupling the complimentary copy to the signal entity.

The signal entity employed may be any chemical, biological or physical entity or particle which is capable of providing a detectable signal or response. Examples of suitable chemical entities include, but are not limited to, small chemical molecules, fluorophores, chemiluminescent tags, or polymers. Polymeric forming chemical reactions or a series of reactions to form a known entity that can be attached and will provide a signal entity can also be employed. Examples of suitable biological entities include, but are not limited to, bacteria, viruses, or the stacking of biological material (for example, the stacking of multiple strands of DNA, or chlorophyll). Examples of suitable physical entities include, but are not limited to, the likes of particles or microbeads. In a preferred embodiment, the signal entity is a physical entity such as coloured microbeads, fluorescent microbeads, magnetic microbeads or light-blocking microbeads. Microbeads formed from polymer materials are preferably employed as they can be readily obtained in a variety of forms, (including coloured, magnetic, and fluorescent), and functionalities (for example, carboxylated or aminated). Examples of suitable polymer microbeads include Polystyrene beads, PMMA beads and PET beads.

Preferably the particles or beads have a dimension of nanometers to millimeters such that the defined areas of the microarray are the same size or smaller than the signal entity. This will allow for a preferred 1:1 ratio between the defined functionalized areas and the particles, irrespective of the number of nucleic acids comprising promoter regions or base sequences bound to the defined functionalized areas, and this will in turn aid in the precise quantification of the number of bound signal entity conjugates used in later sensing applications. The precise quantification also allows the user to ascertain whether there are any non-activated defined areas. Alternatively, the particles or beads may be significantly larger in size than the defined areas of the microarray and may be of any shape.

The number of bound signal entity conjugates is preferably ascertained using the techniques described above in relation to the detecting of target analytes. These techniques include but are not limited to visual (optical) techniques, fluorescent techniques, electrical techniques, magnetic light refraction techniques, heat and frequency responses and digital techniques. Examples of other suitable techniques include, but are not limited to spectrophotometric techniques. The signal entity employed should therefore preferably have specific properties, dependant on the technique employed. For the purposes of this embodiment of the invention and as indicated above, the preferred technique and response is digital, allowing the user to count the number of particles bound.

The signal entity conjugates are preferably washed over the defined functionalized areas of a microarray as an aqueous solution and preferably attach to bound nucleic acids comprising a promoter region or base sequence through a carboxyl or amino group using standard techniques to form a non-covalent double helix. Excess signal entity conjugates are then removed from the surface sites by washing with the carrier solution (typically a buffer) before quantification of the number of bound signal entity conjugates takes place. Detection and quantification of the bound signal entity conjugates can occur by any appropriate technique having regard to the nature of the signal entity to be used. By way of example, if the microarray is mono-functionalized then counting would preferably be achieved by employing light-blocking microbeads such that when light is passed through the microarray the number of bound microbeads can be ascertained by counting the number of defined functionalized areas appear either illuminated or blocked. If the sensor is multi-functionalized then counting would preferably be achieved by employing coloured microbeads, allowing for determination of the numbers of each different colour present on the microarray. The bound signal entity conjugates are then released by a change in the pH of the aqueous solution, and are subsequently employed in the analysis of nucleic acid molecules as indicated above and described below.

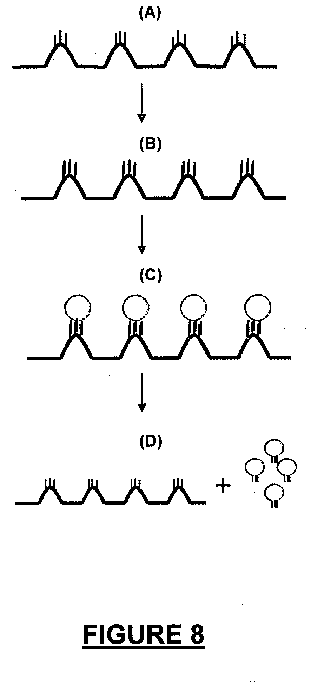

An alternative mthod for functionalizing the defined areas of a two- or three-dimensional microarray, with reference to Figure 8, comprises the following steps:
a) one or more single strands of a nucleic acid comprising a target promoter region or other base sequence of interest is bound to the flat sensor sites or surface structures, i.e. the defined areas of a microarray, through a carboxyl or amino group using standard techniques, including but not limited to DCC coupling (Figure 8, (A));
b) a complimentary strand of the nucleic acid comprising a promoter region or other base sequence of interest (herein defined as the complimentary strand) is synthesized and attached to the bound nucleic acid through complimentary interactions between the carboxyl or amino groups of the nucleic acid strands involved (Figure 8, (B));
c) a signal entity is attached to the complimentary strands to form a signal entity conjugate (Figure 8, (C));
d) the number of bound signal entity conjugates are counted; and
e) the signal entity conjugates are released for use in analysis of nucleic acid molecules, leaving only the nucleic acid(s) bound to the flat sensor sites or surface structures of the microarray (Figure 8, (D)).

As with the previously described method of functionalization, the complimentary strand may be synthesized using a nucleic acid synthesizer (or produced via recombinant techniques, for example) and functionalized with a terminal carboxyl or amino functional group. Likewise, the preferred signal entity is as described above. Preferably, the signal entity conjugate is formed by washing the signal entity over the surface structures of the microarray as an aqueous solution. The signal entities then bind to the functionalized complimentary strands through a carboxyl or amino group, using standard techniques, which include but are not limited to DCC coupling. As for the previously described method of functionalization, the number of bound signal entity conjugates is counted.

Again, functionalizing the microarray and forming a signal entity conjugate in accordance with this alternative method of the present invention where molecules or compounds other than nucleic acid are of interest are broadly described above in relation to the detection of target analytes and skilled persons will readily appreciate appropriate compounds and conditions to be used.

It is preferable that the final step in each of the above functionalization methods, involving the release of the signal entity conjugate, is reversible. This may provide a number of benefits. First, as stated above, the released signal entity conjugates may then be used in the analysis of nucleic acid molecules. Second, it is the release of the signal entity conjugates that allows the microarrays to act as detectors. That is, the inventors have found that functionalizing the defined areas of a two- or three-dimensional microarray with attached nucleic acids comprising a promoter region or base sequence of interest allows for either the subsequent analysis of the presence and/or extent of methylation within a promoter region of a gene or the detection of specific base sequences. Furthermore, reversibility of the final step aids in the quantification of the number of signal entity conjugates used during the sensor application.

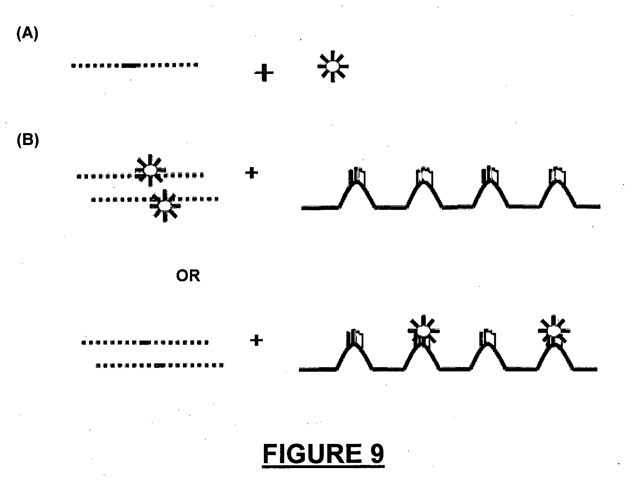

In one embodiment of the present invention, the use of the microarrays as sensors involves running a mixed aqueous sample, containing single strands of nucleic acid from a sample to be analyzed and the signal entity conjugates, which have been released from the array (as described previously), across the microarray (Figure 9). Preferably, and as indicated above, the mixed sample is formed by combining the released signal entity conjugates with the nucleic acid sample in a suitable buffer and at a suitable pH (preferably the pH is 7.0). Preferably, the nucleic acid sample contains the promoter region or other nucleic base sequence of interest.

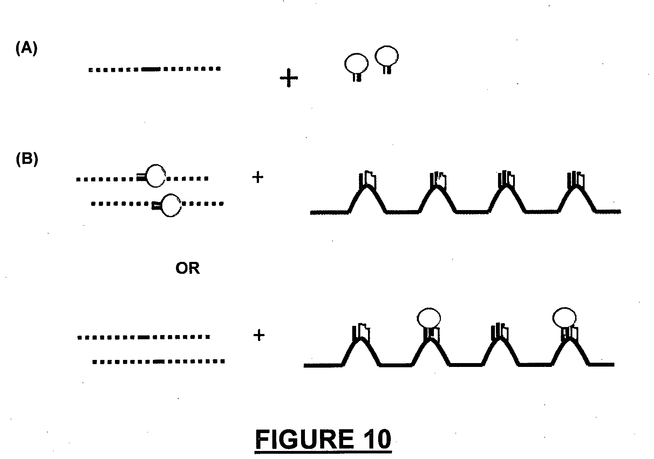

In an alternative embodiment, only unreacted conjugates are run across the microarray (Figure 10). For example, the sample is combined with the signal entity conjugates, signal entity conjugates which do not bind to the sample are separated and run across the microarray.

Within the mixed sample, it is preferable that the signal entity conjugates bind only to base sequences of interest or to methylated Cytosine bases within the promoter regions of interest as they will not have been converted to Uracil during treatment with bisulphite. This selectivity is achieved through interactions between complimentary base pairs. Binding of the signal entity conjugates to the single strands of nucleic acid results in the formation of signal entity conjugate/nucleic acid complexes within the mixed samples. When the functionalized microarray is subsequently exposed to the mixed sample, only those signal entity conjugates which are unbound are available for binding to the functionalized defined areas on the microarray. The user is then able to count the number of signal entity conjugates that bind to the defined areas using the techniques described above. Alternatively, as indicated above, the mixed sample is separated into its component parts, namely the complexes and the free signal entity conjugates, and only the free signal entity conjugates are then run across the defined areas of the microarray. Preferably, the binding of the signal entity conjugates to the functionalized flat sensor sites or surface structures is reversible as this allows the microarrays to be used repeatedly and stored, and preferably this reversibility is achieved by changing the pH of the aqueous solution. Preferably, the microarrays functionalized according to the above methods are stored at about 4°C.

A decrease in the number of un-reacted signal entity conjugates, when compared to the known number of signal entity conjugates as ascertained from the functionalization of the microarray, will signify that a base sequence of interest is present. Likewise, a decrease in the number of un-reacted signal entity conjugates will signify that the promoter region of the gene was methylated to some extent. No change in binding will indicate that either the base sequence of interest was not present or that the promoter region of the gene was not methylated.

It is the qualitative and quantitative nature of the functionalized microarray which therefore allows detection of specific nucleotide sequences or the presence and/or extent of methylation to be determined. The qualitative and quantitative nature of the functionalized microarray also allows the user to determine whether the promoter region for a specific gene is available for transcription. The ability to determine the presence and/or extent of methylation also allows the user to determine the influences of various factors, including but not limited to, environmental factors, diet or medication.

The ability to accurately count the number of signal entity conjugates bound or attached to the surface of the microarray is an important aspect of the present invention because it allows for direct quantification of the number of nucleic acid strands present within a sample. For example, if a promoter region of interest in the sample occurs only once and is methylated then the number of signal entity conjugates bound to the microarray will be equal to the number of nucleic acid strands in the sample. Surprisingly, the inventors have also found that due to the sensitivity of the process the invention obviates the need for amplification of the nucleic acids in a sample using techniques such as PCR, although for certain applications one may still choose to employ an amplification technique. This is a significant aspect of the present invention, as the replication or amplification of nucleic acids is time consuming and can result in a number of errors. Eliminating the need for replication restricts the possibility of errors occurring. Furthermore, the method itself allows the user to determine the concentration of DNA within a sample.

As indicated previously, and using the techniques described previously, increased sensitivity of the defined functionalized areas of the microarray (i.e. flat sites on a two-dimensional microarray or the tips/tops of surface structures on a three-dimensional microarray) is achieved by coating the base material of the three-dimensional microarray with an inert material. This minimises non-specific binding and allows for more accurate measurements such as digital counting (best seen at Figure 1, (C) and (D)).

Finally because microarrays are employed as the sensor platform, this invention also allows for simultaneous assaying of nucleic acids comprising promoter regions, other regions of a nucleic acid, genes and/or genomes (or one or more other compounds). Multiple assays could be carried out using microarrays whose surfaces have either been functionalized in a uniform or random manner with a variety of the appropriate complimentary genes or genomes.

In summary, as a result of being able to attach sensory agents or nucleic acid strands either directly to sensory agents on the surface of a two- or three-dimensional microarray, or indirectly through linker groups, the detection of single molecules and the analysis of nucleic acid molecules can potentially be achieved with high sensitivity due to the large number of individual flat sensor sites or surface structures per cm². Coating the base material of the two- or three-dimensional microarray with an inert material can be preferable to ensure a high degree of sensitivity is achieved and to eliminate non specific binding. Preferably gold is used as the inert coating material.

### Examples

### Example 1: Immobilisation of bead conjugates on the surface of a three-dimensional microarray.

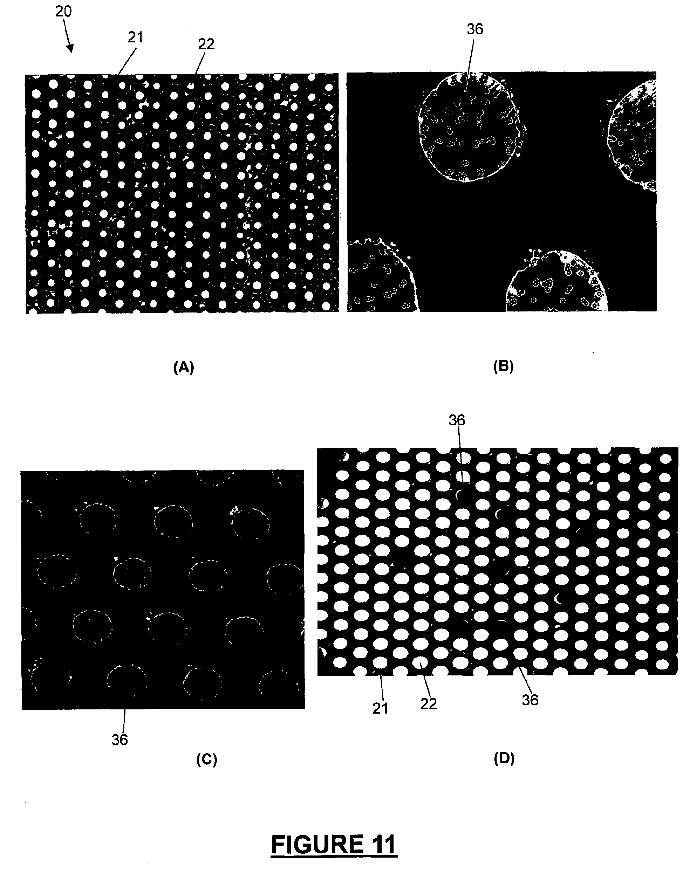

Following the process shown in the diagrammatic depiction in Figure 5, and with reference to Figure 11, the following experiment was carried out using coloured bead conjugates as the target analyte:
A three-dimensional microcone array 20, manufactured from a PMMA polymer substrate and coated in gold 21 was employed (Figure 11, (A)). The cone tips 22 measured 100 µm in diameter. Following removal of gold from the cone tips 22 by abrasion an -NH₂ functional group was attached to the exposed PMMA polymer surface at the defined area formed by the exposed cone tips 22. Thus a defined fuctionalized area at exposed cone tips 22 is formed. This was achieved by exposing the surface of the microarray to 1 to 20% ethylenedamine in DMSO for 5 to 20 minutes. The surface was then washed with IPA and dried under N₂ gas. The surface was then exposed to 2 % GA in sodium carbonate buffer (pH 9.2) for two hours with shaking at room temperature. This was followed by a water wash. Subsequent attachment of a linker group was achieved by exposing the surface of the microarray to 2 % 1,6-hexyldiamine in sodium carbonate buffer (pH 9.2) for two hours with shaking at room temperature, followed by water washes. The NH₂-functionalized surface was then attached by micro-PS-CO₂H beads (8 microns in diameter) in Borate buffer (pH 8.5) after beads activation with EDC and NHS in MES buffer (pH 6.8) (Figure 5). In future, such NH₂-functionalized sensing surface can be used for attachment of a sensory agent (for example, an antibody, DNA, etc). For example, a tip surface NH₂ group can join a protein NH₂ group through a GA linker. This was achieved by loading the sensory agent in a borate buffer (pH 8.5) onto the surface of the microarray and leaving it overnight. A PBS buffer wash was then carried out. The surface was then exposed to microbead conjugates 36 (pictorially represented as 35 in Figure 2, (C), and shown in Figure 11, (B) and (C)) comprising an appropriate binding partner for the sensory agent (for example, antigens, DNA, etc) to test the functional working of the three-dimensional microarray. Digital measurements were carried out to ascertain the extent of binding of the microbead conjugates 36. To achieve this, light was passed through the sample. Where light was blocked, microbead conjugates were attached and their number was able to be digitally counted using a commercial MicroScanner, Microscope or/and Digital image. Figure 11, (D) shows a diagrammatic representation of a digital result showing the blocked areas.

**Examples 2 and 3:** Schemes 1 and 2 in the Examples below show schematic representations of the process described in Examples 2 and 3, respectively. Steps (1) to (4) of Scheme 1 are microarray preparation steps as discussed earlier and are applicable to both examples 2 and 3. A key for components A to G in both Schemes is provided in Scheme 1.

### Example 2: Sandwich assay for large protein molecules

Scheme 1, steps (5) to (7) show a sandwich assay for rat IgG. The three-dimensional microarray employed was 100 µm in diameter and was formed from PMMA substrates. After removing gold from the cone tips and introducing a -NH₂ functional group to each cone tip, the cone tips were further reacted with glutaraldehyde in PBS buffer (pH 7.4) to bind a -CHO functional group to the functionalized cone tips. A solution of commercial *anti*-rat IgG (secondary antibody) (R5128, Sigma) in PBS was then reacted with the cone tips overnight to immobilize the *anti*-rat IgG onto the tips. After washing the microarray with PBS, a solution of commercial proteins (Rat IgG) (P1922, Sigma) in PBS and in various concentrations, are reacted with the *anti*-rat IgG on the tips at 40 °C for 1 hour. Finally, after a PBS wash of the microarray, a suspension of *anti*-rat IgG coated microparticles was further reacted with the substrates at 40 °C for 1 hour to attach the microbeads to the cone tips. After PBS washing and gently drying the substrates, the concentration of the protein analyte (Rat IgG) was proportional to the numbers of microbeads attached on the tips, which were then able to be digitally counted using a commercial MicroScanner, Microscope and/or Digital image.

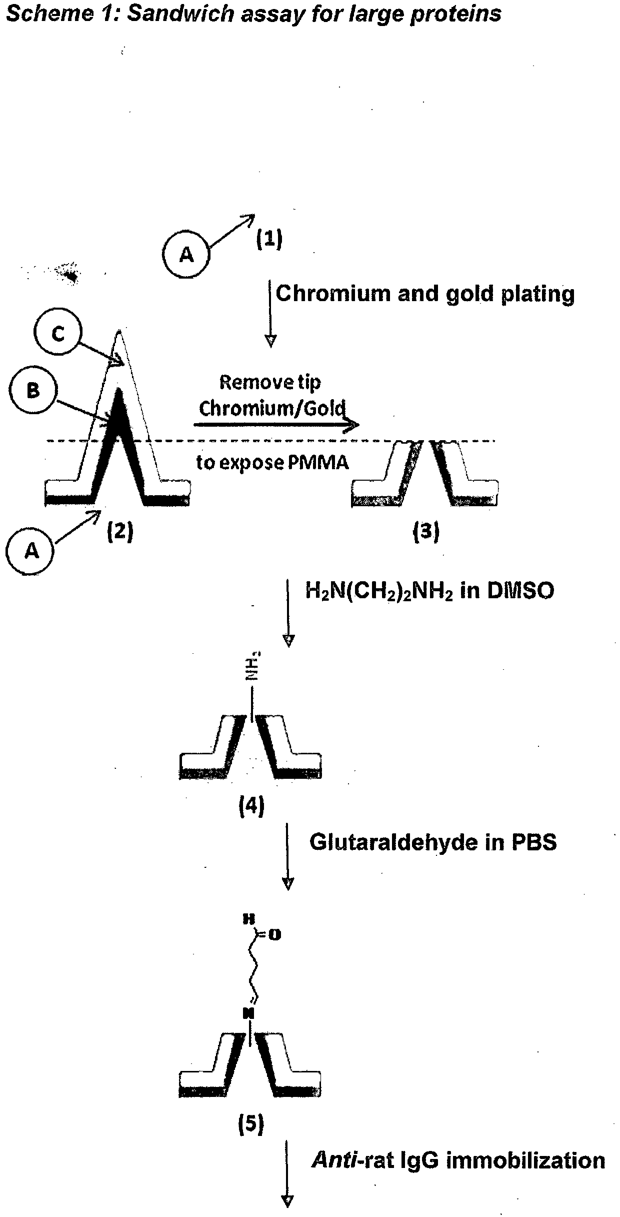

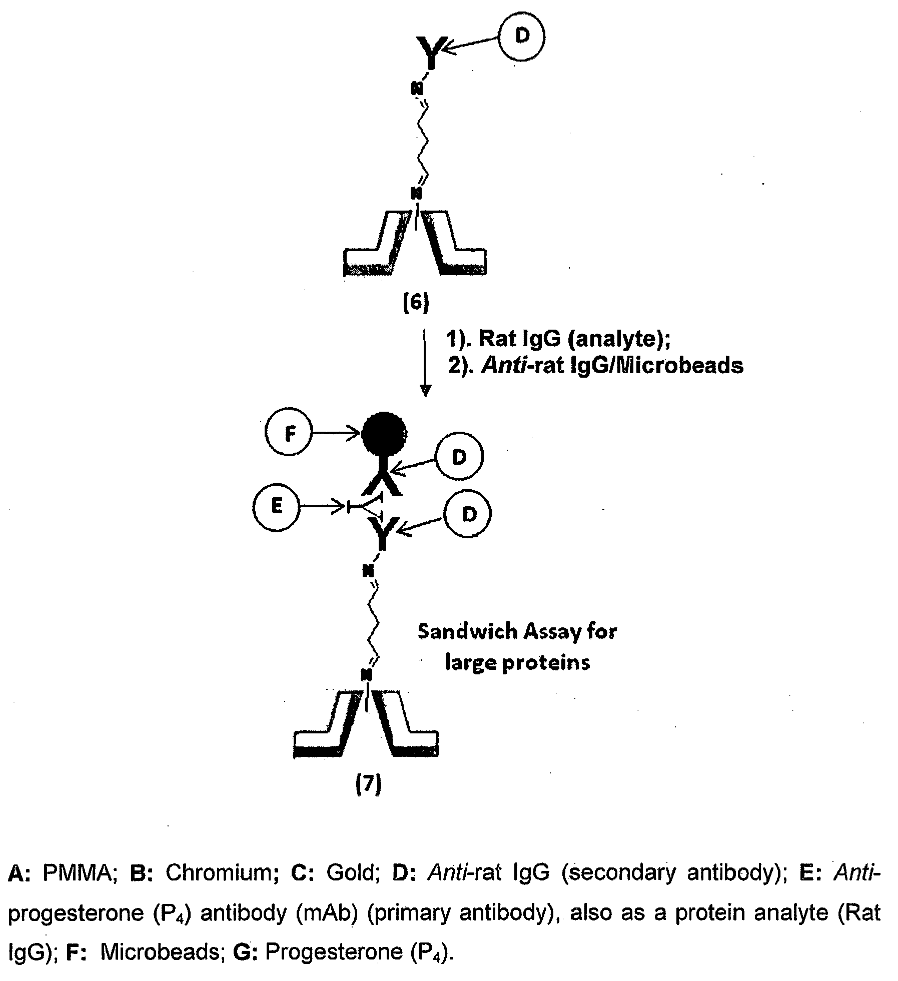

### Example 3: Inhibition assay for small steroid molecules

Scheme 1, steps (5), (8) and (9) shows an inhibition assay for small molecules. Following introduction of -CHO functional groups to the cone tips of three-dimensional microcone array (100 µm in diameter) PMMA substrates, a solution of a Progesterone-PEG-NH₂ (P₄-PEG-NH₂), prepared according to a literature method (J. S. Mitchell et al, *Analytical Biochemistry*, **2005, 343**:125-135), in PBS (pH 7.4) was reacted with the cone tips overnight for immobilization of steroid progesterone (P₄) on the microcone tips. After washing the substrates with PBS, a fixed amount of the commercial monoclonal *anti*-progesterone antibody (mAb, primary antibody) (P1922, Sigma) was mixed with various standard progesterone (P₄) solutions in PBS (pH 7.4) at 40 °C for 1 hour. The resulting mixture was then bound to the Progesterone (P₄) attached to the tips. This reaction was carried out at 40 °C for 1 hour. Finally, after a PBS wash of the substrates, a suspension of *anti*-rat IgG (secondary antibody) coated microparticles was further reacted with the substrates at 40 °C for 1 hour to attach the microbeads to the cone tips. After PBS washing and gently drying the substrates, the concentration of the steroid (progesterone) was reverse proportional to the numbers of microbeads attached on the tips, which were then able to be digitally counted using a commercial MicroScanner, Microscope and/or Digital image.

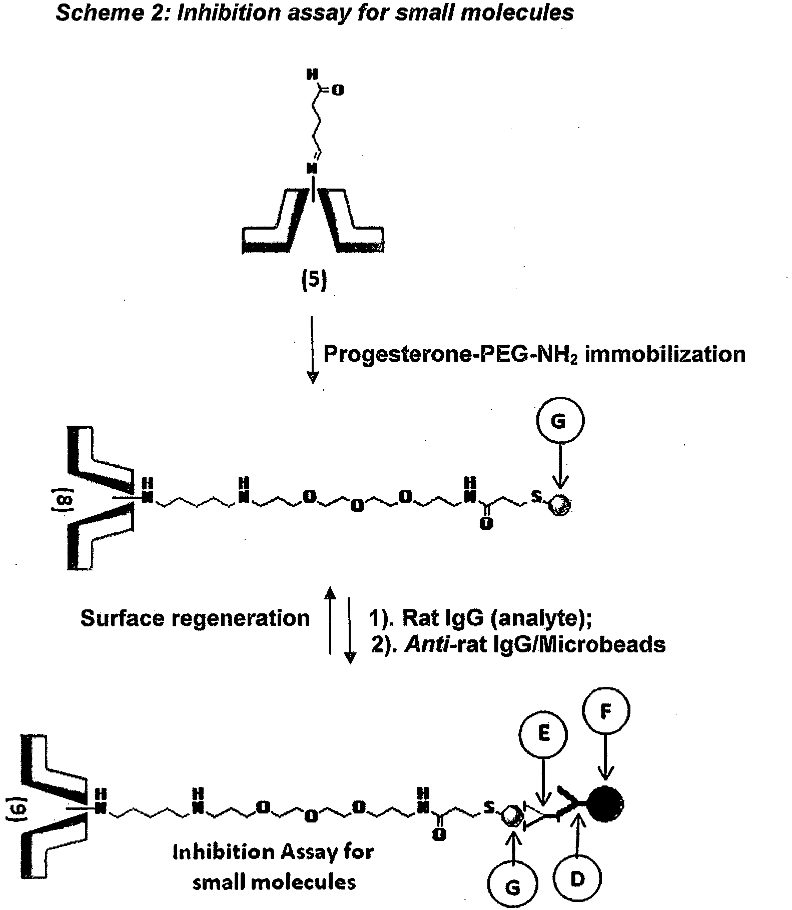

### Example 4: DNA hybridizations

Scheme 3, shown below, is a schematic representation of the process described in Example 4. Scheme 2 provides an alternative route from step (5) shown in schemes 1 and 2 via new steps (10) to (12). The key provided for Scheme 1 is relevant to Scheme 3.

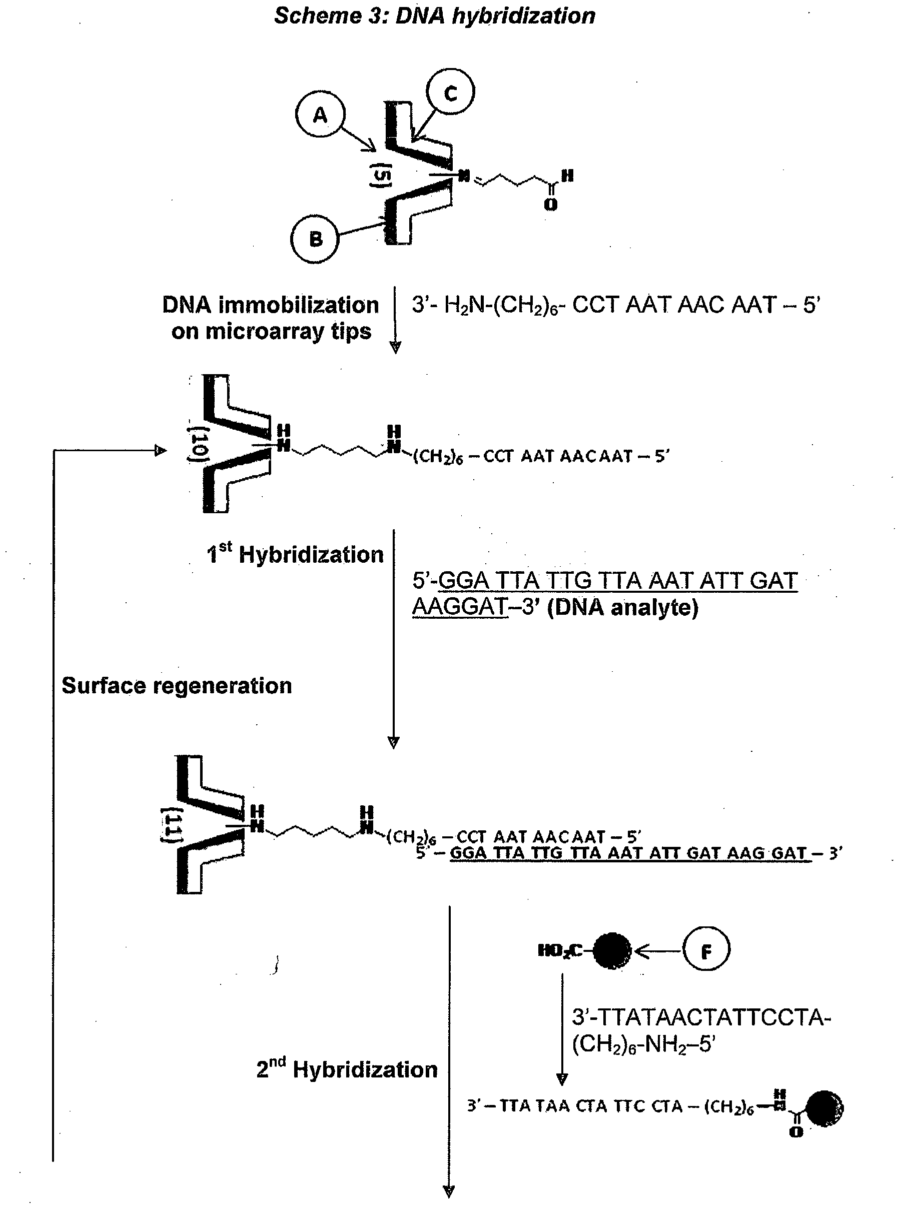

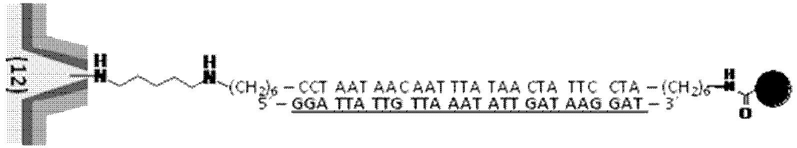

After -CHO functionalization of the three-dimensional microcone tips, a H₂O wash and N₂ drying, a solution of synthetic amine-attached 12-base oligonucleotides of a given sequence [H₂N-(CH₂)₆-CCTAATAACAAT] in phosphate buffer (pH 7.0) was immobilized on the microcone tips overnight, followed by washings with 5XSSC, H₂O and N₂ drying.

Prior to hybridization, glutaraldehyde-activated substrates were treated with Na₂BH₄. The first hybridization was carried out as follows: 12-base DNA immobilized microcone tips were hybridized with a synthetic target DNA (27-base, 5'-GGATTATTGTTAAATATTGATAAGGAT-3') in a PBS hybridization buffer (8 x PBS, pH7.0) for 24 hours, followed by 2 x SSC washes. The second hybridization was performed by a further reaction of the hybridized microcone tips with DNA attached microparticles [15-base, 3'-TTATAACTATTCCTA-(CH₂)₆-NHCO-Microparticle] in the same hybridization buffer (8 x PBS) for 5 to 24 hours. Finally, the microparticles-attached substrates were washed with 8 x PBN buffer (0.3M NaNO3 and PBS, pH7.0) several times. The quantification of target DNA (27-base) was also carried out by digitally counting using a commercial MicroScanner, Microscope and/or Digital image. The concentration of the target DNA was proportional to the microparticles on the microcone tips of the substrate.

Because microarrays are employed as the sensor platform, this invention also allows for simultaneous assaying of nucleic acids comprising promoter regions, other regions of a nucleic acid, genes and/or genomes (or one or more other compounds). Multiple assays could be carried out using microarrays whose surfaces have either been functionalized in a uniform or random manner with a variety of the appropriate complementary genes or genomes.

As discussed previously, sensory agents, which specifically bind to the target analyte the user wishes to detect, are usually employed. To prevent non-specific binding to the coated surface the microarray is exposed to 2 % OVA in PBS for 2 hours at room temperature with shaking. This is followed by a PBS wash. The microarray is then ready to use.

Because microarrays are employed as the sensor platform, this invention also allows for simultaneous assaying of nucleic acids comprising promoter regions, other regions of a nucleic acid, genes and/or genomes (or one or more other compounds). Multiple assays could be carried out using microarrays whose surfaces have either been functionalized in a uniform or random manner with a variety of the appropriate complimentary genes or genomes.

The foregoing describes the invention including preferred forms thereof. Modifications and alterations that would be readily apparent to the skilled person are intended to be included within the scope of the invention described.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to."

## Claims

1. A three-dimensional microarray for use in determining the presence of a target compound(s) of interest within a sample, the microarray including:
a) a base material including a plurality of surface structures forming part of and protruding from the base material;
b) the plurality of surface structures including sensory agent(s) capable of attachment to the target compound(s) of interest within the sample, the sensory agent(s) being included on defined functionalized areas on the tops of each surface structure; and
wherein the microarray is coated between the defined functionalized areas with an inert material to minimize non-specific binding.

2. The three-dimensional microarray of claim 1 wherein the surface structures take the form of cones or ridges.

3. The three-dimensional microarray of claim 2, wherein the defined functionalized areas of each surface structure is the tip of said cones or ridges.

4. The three-dimensional microarray of claim 1 or 2 wherein there are between about 250,000 and about 1 billion defined functionalized areas per cm².

5. The three-dimensional microarray of any one of claims 1, 2 or 4 wherein the microarray is formed from a plastics material, a metal, a ceramic, an oxide, silicon, a photoresist or a polymer substrate.

6. The three-dimensional microarray of any one of claims 1, 2, 4 or 5 wherein a patterned layer is attached to or formed onto the underside of the base material to disperse light across the surface where light is passed through the microarray for measurement purposes.

7. The three-dimensional microarray of any one of claims 1, 2 or 4 to 6 wherein the surface structures are formed by etching, lithographic processes, hot embossing, nano-embossing, injection molding or by the Continuous Forming Technology process.

8. The three-dimensional microarray of any one of claims 1, 2 or 4 to 7 wherein the sensory groups are one or more single strands of nucleic acid comprising a promoter region or one more single strands of nucleic acid comprising a specific base sequence of interest.

9. The three-dimensional microarray of any one of claims 1, 2 or 4 to 8 wherein the sensory agents are selected to attach to the target compounds as part of antibody/antigen, DNA/DNA, DNA/protein, protein/protein, protein/receptor, cell/protein and cell/DNA binding partners.

10. The three-dimensional microarray of any one of claims 1, 2 or 4 o 9 wherein the microarray includes a layer of a thiol, protein or PEG material beneath the inert coating.

11. The three-dimensional microarray of any one of claims 1, 2 or 4 to 10 wherein the inert material is applied using evaporation, painting, deposition, sputtering, plasma treatment, spray coating, dip coating, or spin coating.

12. A method for determining the presence of a target compound(s) of interest within a sample, the method including the steps of:
a) providing the three-dimensional microarray of any one of claims 1-11;
b) passing at least part of the sample over the microarray; and
c) determining the presence of a target compound(s) of interest by detection of a detectable response to the attachment of the target compound(s) to the sensory agent(s).

13. The method of claim 12 wherein the detectable response is selected from colour, fluorescence, light blocking, visual responses, spectrophotometric responses, potentiometric or galvanostatic responses, magnetic light refraction, heat, frequency and digital responses.

14. The method of claim 12 or 13 wherein the detectable response is capable of being read by digital counting, weight measurements, fluorescence, optical, and/or electrical means.

15. The method of any one of claims 12 to 14 wherein the detectable response results in any one or a combination of quantitative/qualitative, fluorescence, optical or colourmetric measurements.

16. A method for determining whether or not a nucleic acid comprising a specific sequence of bases is present in a sample, the method comprising:
a) in a sample of nucleic acid, where the nucleic acid is double stranded, separating it into single strands;
b) combining the single strands of a nucleic acid with a signal entity conjugate to form a mixed sample;
c) determining whether the nucleic acid comprising a specific sequence of bases is present by running the mixed sample across the surface of a functionalized microarray according to any one of claims 1 to 11; and
d) counting the number of bound signal entity conjugates.

17. A method for determining the extent of methylation in the promoter region of a gene, the said method comprising:
a) in a sample of nucleic acid, where the nucleic acid is double stranded, separating it into single strands;
b) treating the sample of nucleic acid such that non-methylated Cytosine is converted to Uracil;
c) combining the single strands of nucleic acid with a signal entity conjugate to form a mixed sample;
d) determining the presence and/or extent of methylation in the promoter regions by running the mixed sample across the surface of a functionalized microarray according to any one of claims 1 to 11; and
e) counting the number of bound signal entity conjugates.

## Patentansprüche

1. Dreidimensionales Mikroarray für die Verwendung zum Bestimmen des Vorhandenseins einer Zielverbindung/von Zielverbindungen von Interesse in einer Probe, wobei das Mikroarray umfasst:
a) ein Basismaterial, das eine Vielzahl von Oberflächenstrukturen umfasst, die einen Teil des Basismaterials bilden und davon vorragen;
b) wobei die Vielzahl von Oberflächenstrukturen (ein) sensorische(s) Mittel umfasst, das/die zum Anlagern an die Zielverbindung(en) von Interesse in der Probe fähig ist/sind, wobei das/die sensorische(n) Mittel an definierten funktionalisierten Bereichen an der Oberseite jeder Oberflächenstruktur enthalten ist/sind; und
wobei das Mikroarray zwischen den definierten funktionalisierten Bereichen mit einem inerten Material beschichtet ist, um unspezifisches Binden zu minimieren.

2. Dreidimensionales Mikroarray gemäß Anspruch 1, wobei die Oberflächenstrukturen die Form von Kegeln oder Rippen annehmen.

3. Dreidimensionales Mikroarray gemäß Anspruch 2, wobei die definierten funktionalisierten Bereiche jeder Oberflächenstruktur die Spitzen der Kegel oder Rippen sind.

4. Dreidimensionales Mikroarray gemäß Anspruch 1 oder 2, wobei zwischen etwa 250.000 und etwa 1 Milliarde definierte funktionalisierte Bereiche pro cm² vorliegen.

5. Dreidimensionales Mikroarray gemäß einem der Ansprüche 1, 2 oder 4, wobei das Mikroarray aus einem Kunststoffmaterial, einem Metall, einer Keramik, einem Oxid, Silicium, einem Photoresist oder einem Polymersubstrat besteht.

6. Dreidimensionales Mikroarray gemäß einem der Ansprüche 1, 2, 4 oder 5, wobei eine strukturierte Schicht an der Unterseite des Basismaterials angebracht oder daran gebildet ist, um Licht über die Oberfläche zu verteilen, wo Licht für Messzwecke durch das Mikroarray geleitet wird.

7. Dreidimensionales Mikroarray gemäß einem der Ansprüche 1, 2 oder 4 bis 6, wobei die Oberflächenstrukturen durch Ätzen, photolithographische Verfahren, Heißprägen, Nanoprägen, Spritzguss oder durch das Continuous-Forming-Technology-Verfahren gebildet sind.

8. Dreidimensionales Mikroarray gemäß einem der Ansprüche 1, 2 oder 4 bis 7, wobei die sensorischen Gruppen an einem oder mehreren Nukleinsäure-Einzelsträngen vorliegen, die einen Promoterbereich umfassen, oder einem oder mehreren Nukleinsäure-Einzelsträngen, die eine spezifische Basensequenz von Interesse umfassen.

9. Dreidimensionales Mikroarray gemäß einem der Ansprüche 1, 2 oder 4 bis 8, wobei die sensorischen Mittel ausgewählt sind, um sich an die Zielverbindungen als Teil von Antikörper/Antigen-, DNA/DNA-, DNA/Protein-, Protein/Protein-, Protein/Rezeptor-, Zelle/Protein- und Zelle/DNA-Bindungspartnern anzulagern.

10. Dreidimensionales Mikroarray gemäß einem der Ansprüche 1, 2 oder 4 bis 9, wobei das Mikroarray eine Schicht aus einem Thiol-, Protein- oder PEG-Material unterhalb der inerten Beschichtung umfasst.

11. Dreidimensionales Mikroarray gemäß einem der Ansprüche 1, 2 oder 4 bis 10, wobei das inerte Material unter Verwendung von Aufdampfen, Aufstreichen, Abscheiden, Sputtering, Plasmabehandlung, Sprühbeschichtung, Tauchbeschichtung oder Aufschleudern aufgebracht ist.

12. Verfahren zum Bestimmen des Vorhandenseins einer Zielverbindung/von Zielverbindungen von Interesse in einer Probe, wobei das Verfahren die Schritte umfasst:
a) Bereitstellen des dreidimensionalen Mikroarrays gemäß einem der Ansprüche 1-11;
b) Leiten wenigstens eines Teils der Probe über das Mikroarray; und
c) Bestimmen des Vorhandenseins einer Zielverbindung/von Zielverbindungen von Interesse durch Nachweisen einer nachweisbaren Antwort auf die Anlagerung der Zielverbindung(en) an das/die sensorische(n) Mittel.

13. Verfahren gemäß Anspruch 12, wobei die nachweisbare Antwort ausgewählt ist aus Farbe, Fluoreszenz, Lichtblockierung, visuellen Antworten, spektrophotometrischen Antworten, potentiometrischen oder galvanostatischen Antworten, magnetischer Lichtbrechung, Wärme, Frequenz und digitalen Antworten.

14. Verfahren gemäß Anspruch 12 oder 13, wobei die nachweisbare Antwort geeignet ist, durch digitales Auszählen, Gewichtsmessungen, Fluoreszenz, optische und/oder elektrische Mittel ausgelesen zu werden.

15. Verfahren gemäß einem der Ansprüche 12 bis 14, wobei die nachweisbare Antwort zu einem oder einer Kombination von quantitativen/qualitativen, Fluoreszenz-, optischen oder kolorimetrischen Messungen führt.

16. Verfahren zum Bestimmen, ob eine Nukleinsäure, die eine spezifische Sequenz von Basen umfasst, in einer Probe vorhanden ist oder nicht, wobei das Verfahren umfasst:
a) in einer Nukleinsäureprobe, wenn die Nukleinsäure doppelsträngig ist, Trennen davon zu Einzelsträngen;
b) Kombinieren der Einzelstränge einer Nukleinsäure mit einem Signaleinheit-Konjugat, um eine gemischte Probe zu bilden;
c) Bestimmen, ob die Nukleinsäure, die eine spezifische Sequenz von Basen umfasst, vorhanden ist, indem die gemischte Probe über die Oberfläche eines funktionalisierten Mikroarrays gemäß einem der Ansprüche 1 bis 11 geleitet wird; und
d) Zählen der Zahl von gebundenen Signaleinheit-Konjugaten.

17. Verfahren zum Bestimmen des Ausmaßes von Methylierung in dem Promoterbereich eines Gens, wobei das Verfahren umfasst:
a) in einer Nukleinsäureprobe, wenn die Nukleinsäure doppelsträngig ist, Trennen davon zu Einzelsträngen;
b) Behandeln der Probe von Nukleinsäure, so dass nichtmethyliertes Cytosin zu Uracil umgewandelt wird;
c) Kombinieren der Einzelstränge von Nukleinsäure mit einem Signaleinheit-Konjugat, um eine gemischte Probe zu bilden;
d) Bestimmen des Vorhandenseins und/oder Ausmaßes von Methylierung in den Promoterbereichen, indem die gemischte Probe über die Oberfläche eines funktionalisierten Mikroarrays gemäß einem der Ansprüche 1 bis 11 geleitet wird; und
e) Zählen der Zahl von gebundenen Signaleinheit-Konjugaten.

## Revendications

1. Microréseau tridimensionnel destiné à être utilisé en détermination de la présence d'un ou plusieurs composés cibles présentant de l'intérêt à l'intérieur d'un échantillon, le microréseau comprenant:
a) un matériau de base comprenant une pluralité de structures de surface faisant partie du matériau de base et faisant saillie de celui-ci ;
b) la pluralité de structures de surface comprenant un ou plusieurs agents de détection pouvant se fixer au ou aux composés cibles présentant de l'intérêt à l'intérieur de l'échantillon, le ou les agents de détection étant inclus sur des zones fonctionnalisées définies sur les parties supérieures de chaque structure de surface ; et
le microréseau étant revêtu entre les zones fonctionnalisées définies d'un matériau inerte pour réduire au minimum la liaison non spécifique.

2. Microréseau tridimensionnel selon la revendication 1, dans lequel les structures de surface prennent la forme de cônes ou de nervures.

3. Microréseau tridimensionnel selon la revendication 2, dans lequel les zones fonctionnalisées définies de chaque structure de surface sont la pointe desdits cônes ou nervures.

4. Microréseau tridimensionnel selon la revendication 1 ou 2, dans lequel il y a entre environ 250 000 et environ 1 milliard de zones fonctionnalisées définies par cm².

5. Microréseau tridimensionnel selon l'une quelconque des revendications 1, 2 ou 4, le microréseau étant formé à partir d'une matière plastique, d'un métal, d'une céramique, d'un oxyde, de silicium, d'une résine photosensible ou d'un substrat en polymère.

6. Microréseau tridimensionnel selon l'une quelconque des revendications 1, 2, 4 ou 5, dans lequel une couche à motif est fixée à la face inférieure du matériau de base ou formée sur celle-ci pour disperser de la lumière sur l'ensemble de la surface quand de la lumière est amenée à passer à travers le microréseau à des fins de mesure.

7. Microréseau tridimensionnel selon l'une quelconque des revendications 1, 2 ou 4 à 6, dans lequel les structures de surface sont formées par gravure, des processus lithographiques, impression en relief à chaud, nano-impression en relief, moulage par injection ou par le processus de technologie de formage en continu.

8. Microréseau tridimensionnel selon l'une quelconque des revendications 1, 2 ou 4 à 7, dans lequel les groupes de détection sont un ou plusieurs simples brins d'acide nucléique comprenant une région promoteur ou un ou plusieurs simples brins d'acide nucléique comprenant une séquence de bases présentant de l'intérêt particulière.

9. Microréseau tridimensionnel selon l'une quelconque des revendications 1, 2 ou 4 à 8, dans lequel les agents de détection sont choisis pour se fixer aux composés cibles en tant que partie de partenaires de liaison anticorps/antigène, ADN/ADN, ADN/protéine, protéine/protéine, protéine/récepteur, cellule/protéine et cellule/ADN.

10. Microréseau tridimensionnel selon l'une quelconque des revendications 1, 2 ou 4 à 9, le microréseau comprenant une couche d'une substance thiol, protéine ou PEG au-dessous du revêtement inerte.

11. Microréseau tridimensionnel selon l'une quelconque des revendications 1, 2 ou 4 à 10, dans lequel le matériau inerte est appliqué à l'aide d'une évaporation, d'une peinture, d'un dépôt, d'une pulvérisation cathodique, d'un traitement par plasma, d'un revêtement par pulvérisation, d'un revêtement par immersion ou d'un dépôt à la tournette.

12. Procédé permettant de déterminer la présence d'un ou plusieurs composés cibles présentant de l'intérêt à l'intérieur d'un échantillon, le procédé comprenant les étapes consistant à:
a) utiliser le microréseau tridimensionnel selon l'une quelconque des revendications 1-11 ;
b) faire passer au moins une partie de l'échantillon sur le microréseau ; et
c) déterminer la présence d'un ou plusieurs composés cibles présentant de l'intérêt par détection d'une réponse détectable à la fixation du ou des composés cibles au ou aux agents de détection.

13. Procéder selon la revendication 12, dans lequel la réponse détectable est choisie parmi une couleur, la fluorescence, le blocage de lumière, les réponses visuelles, les réponses spectrophotométriques, les réponses potentiométriques ou galvanostatiques, la réfraction de lumière magnétique, de la chaleur, une fréquence et les réponses numériques.

14. Procédé selon la revendication 12 ou 13, dans lequel la réponse détectable peut être lue par comptage numérique, des mesures de poids, fluorescence, un moyen optique et/ou électrique.

15. Procédé selon l'une quelconque des revendications 12 à 14, dans lequel la réponse détectable résulte en une mesure quelconque parmi les mesures quantitatives/qualitatives, de fluorescence, optiques ou colorimétriques ou une association de celles-ci.

16. Procédé permettant de déterminer si un acide nucléique comprenant une séquence de bases particulière est présent dans un échantillon ou non, le procédé consistant à:
a) dans un échantillon d'acide nucléique, quand l'acide nucléique est double brin, le séparer en simples brins ;
b) combiner les simples brins d'un acide nucléique avec un conjugué d'entité de signal pour former un échantillon mélangé;
c) déterminer si l'acide nucléique comprenant une séquence de bases particulière est présent en faisant passer l'échantillon mélangé sur l'ensemble de la surface d'un microréseau fonctionnalisé selon l'une quelconque des revendications 1 à 11 ; et
d) compter le nombre de conjugués d'entité de signal liés.

17. Procédé permettant de déterminer le degré de méthylation dans la région promoteur d'un gène, ledit procédé consistant à:
a) dans un échantillon d'acide nucléique, quand l'acide nucléique est double brin, le séparer en simples brins ;
b) traiter l'échantillon d'acide nucléique de façon telle que la cytosine non méthylée est convertie en uracile ;
c) combiner les simples brins d'acide nucléique avec un conjugué d'entité de signal pour former un échantillon mélangé;
d) déterminer la présence et/ou le degré de méthylation dans les régions promoteurs en faisant passer l'échantillon mélangé sur l'ensemble de la surface d'un microréseau fonctionnalisé selon l'une quelconque des revendications 1 à 11 ; et
e) compter le nombre de conjugués d'entité de signal liés.
